# EUROPEAN PATENT APPLICATION

(11) **EP 4 596 685 A1**
(43) Date of publication of application: **06.08.2025**
(21) Application number: 23872630.1
(22) Date of filing: 29.09.2023
(51) Int. Cl.: C12N 5/10, A61K 35/17, A61P 35/00, C12N 5/0783, C12N 15/12, C12N 15/24, C12N 15/62, C12N 15/85

(54) **IMMUNE CELL INDUCED FROM IPS CELL**

(30) Priority: 30.09.2022 JP 2022157256
(71) Applicant: Kyoto University, Kyoto-shi, Kyoto 606-8501 (JP)
(72) Inventor: KANEKO Shin, Kyoto-shi, Kyoto 606-8501 (JP); ISHIKAWA Akihiro, Kyoto-shi, Kyoto 606-8501 (JP); KANIE Keitaro, Kyoto-shi, Kyoto 606-8501 (JP)
(74) Representative: Hoffmann Eitle
(86) International application number: PCT/JP2023/035752
(87) International publication number: WO 2024/071411

(57) **Abstract**

To provide an immune cell induced from an iPS cell provided with a phenotype advantageous for migration and infiltration into solid tumor sites, and for proliferation and survival of the immune cell in the solid tumor. The present invention provides an immune cell induced from an iPS cell, and expressing a cell surface molecule reactive to a tumor-related antigen, and interleukin 15. The invention further provides an immune cell induced from an iPS cell, and expressing a cell surface molecule reactive to a tumor-related antigen, and interleukin 12, interleukin 18, or interleukin 21.

## Description

### Technical Field

The present invention relates to an immune cell induced from an iPS cell (induced pluripotent stem cell), wherein the immune cell expresses a cell surface molecule reactive to a tumor-related antigen and interleukin 15 (IL-15), a method for producing the immune cell, a pharmaceutical containing the immune cell, a cytotoxic agent for a cell expressing a tumor-related antigen and an agent for preventing or treating cancer in a mammal, the agents containing the immune cell, and a method for preventing or treating cancer in a mammal, including administering an effective amount of the immune cell.

### Background Art

In recent years, cancer immunotherapy for eliminating cancer by activating immune function within the body of a patient is being actively researched as novel cancer treatment. In the cancer immunotherapy, a T cell specifically recognizing and attacking cancer is significant. In the body of a patient, however, the amount of the T cell recognizing cancer is small. Therefore, cancer immunotherapy using a genetically modified T cell that expresses, on a cell surface, a receptor capable of specifically recognizing a cancer antigen or a tumor-related antigen has been researched. As the genetically modified T cell, T cells expressing a chimeric antigen receptor (CAR) targeting a specific cancer antigen or tumor-related antigen, and expressing a T cell receptor (TCR) have been reported, and these are respectively designated as a CAR-T cell and a TCR-T cell (Patent Literatures 1 and 2).

A CAR is a fusion protein of an antigen recognition site of an antibody specifically recognizing a cancer antigen or a tumor-related antigen, and an intracellular domain derived from a TCR, and a CAR-T cell can recognize an antigen expressed on a cell surface without being constrained by a human leukocyte antigen (HLA). A TCR is a receptor used when a T cell recognizes an antigen, and is constituted by a dimer consisting of an α chain and a β chain, or a γ chain and a δ chain. A TCR forms a complex together with a CD3 molecule group on a T cell surface, and activates the T cell by recognizing an antigen molecule bound to a major histocompatibility complex (MHC) molecule.

In the cancer immunotherapy using the genetically modified T cell, difficulty in ensuring a sufficient amount of T cells, and T cell exhaustion including deterioration of T cell proliferation and deterioration of immune reaction against an antigen such as a target cell are obstacles in effectively performing the cancer immunotherapy. For overcoming the obstacles to smoothly perform the cancer immunotherapy, a technique has been reported in which an induced pluripotent stem cell (iPS cell) established from an antigen-specific T cell is proliferated, and then differentiated into a cytotoxic T cell (Non Patent Literature 1). It is presumed that this technique can be a significant base of the cancer immunotherapy using a T cell because a large number of functional T cells can be continuously induced from iPS cells by this technique.

The cancer immunotherapy using a CAR-T cell has provided a dramatic effect of, for example, achieving complete remission in some patients of blood tumor. The cancer immunotherapy using the genetically modified T cell has not, however, always shown a satisfactory therapeutic effect against solid tumor (Non Patent Literature 2). It has been reported that it is significant, for the genetically modified T cell to exhibit an antitumor effect on solid tumor, that the T cell retaining the function migrates and infiltrates into the solid tumor to survive in the tumor for a long period of time to continuously exhibit cytotoxic activity (Non Patent Literature 3).

As one method for causing the genetically modified T cell to exhibit sufficient antitumor effect on solid tumor, gene transfer, into a T cell, of a factor for improving the function of the T cell has been proposed. It has been disclosed that, for example, a CAR-T cell in which interleukin 7 (IL-7) that is a cytokine playing a significant role in survival and proliferation of a T cell, and CCL19 that is a chemokine playing a significant role in migration of a T cell and a dendritic cell are expressed by gene transfer is effective for solid tumor (Patent Literature 3). In addition, it has been reported that a CAR-T cell in which interleukin 15 (IL-15) and interleukin 21 (IL-21) that accelerate survival, proliferation and expression of cytotoxic activity of a T cell for improving an antitumor property of the T cell are expressed by gene transfer is effective for solid tumor (Non Patent Literature 4).

### Citation List

### Patent Literature

Patent Literature 1: International Publication No. WO2013/070468
Patent Literature 2: International Publication No. WO2015/173112
Patent Literature 3: International Publication No. WO2017/159736

### Non Patent Literature

Non Patent Literature 1: Minagawa A, et al., Enhancing T cell receptor stability in rejuvenated iPSC-derived T cells improves their use in cancer immunotherapy, Cell Stem Cell, 2018; 23: 850-858
Non Patent Literature 2: Hartmann J., et al., Clinical development of CAR T cells-challenges and opportunities in translating innovative treatment concepts, EMBO Mol Med. 2017; 9: 1183-1197
Non Patent Literature 3: Chen D. S. and Mellman I., Oncology Meets Immunology: The Cancer-Immunity Cycle, Immunity, 2013; 39: 1-10
Non Patent Literature 4: Batra S. A., et al., Glypican-3-specific CAR T cells coexpressing IL15 and IL21 have superior expansion and antitumor activity against hepatocellular carcinoma, Cancer Immunol Res. 2020; 8: 309-320

### Summary of Invention

### Technical Problem

A functional cytotoxic T cell can be induced to differentiate from an iPS cell. An iPS cell can be easily genetically engineered, and is easily evaluated for safety because it is a clone cell. Therefore, in cancer immunotherapy using this type of T cell, use of an iPS cell enables "off-the-shelf" cell treatment, and enables more stable supply of cells for the treatment. Besides, since many of cancers are solid tumors, it is significant to impart sufficient antitumor effect on solid tumor to a T cell or a natural killer (NK) cell differentiated from an iPS cell (iPS-T cell or iPS-NK cell) for improving the treatment result of the cancer immunotherapy using such an immune cell differentiated from an iPS cell.

An object of the present invention is, for obtaining the above-described immune cell excellent in the antitumor effect on solid tumor, to provide an immune cell induced from an iPS cell, provided with a phenotype advantageous to migration and infiltration into the solid tumor site, and to proliferation and survival of the immune cell in the solid tumor. Another object of the present invention is to provide a method for producing the immune cell, a pharmaceutical containing the immune cell, a cytotoxic agent for a cell expressing a tumor-related antigen, containing the immune cell, a method for preventing or treating cancer in a mammal, including administering an effective amount of the immune cell, and an agent for preventing or treating cancer in a mammal, containing the immune cell.

### Solution to Problem

The present inventors have searched for cytokines that improve the function of an immune cell induced from an iPS cell. As a result, it has been found that interleukin 15 (IL-15), or a combination of IL-15 with interleukin 12 (IL-12), interleukin 18 (IL-18) or interleukin 21 (IL-21) improves the function of the immune cell, resulting in accomplishing the present invention.

Specifically, the present invention provides the following:
[1] An immune cell induced from an iPS cell, wherein the immune cell expresses a cell surface molecule reactive to a tumor-related antigen, and interleukin 15 (IL-15).
[2] The immune cell according to [1], wherein the immune cell further expresses interleukin 12 (IL-12), interleukin 18 (IL-18), or interleukin 21 (IL-21).
[3] The immune cell according to [1], wherein the immune cell comprises a nucleic acid encoding the cell surface molecule and a nucleic acid encoding IL-15, introduced extracellularly.
[4] The immune cell according to [3], wherein the immune cell further comprises a nucleic acid encoding IL-12, IL-18, or IL-21, introduced extracellularly.
[5] The immune cell according to [1], wherein the immune cell comprises an expression vector containing a nucleic acid encoding the cell surface molecule, and a nucleic acid encoding IL-15.
[6] The immune cell according to [5], wherein the immune cell further comprises an expression vector containing a nucleic acid encoding IL-12, IL-18, or IL-21.
[7] The immune cell according to [1], wherein the immune cell is a T cell or an NK cell.
[8] The immune cell according to [7], wherein the T cell is a CD8 single positive cytotoxic T cell.
[9] The immune cell according to [1], wherein the cell surface molecule is a chimeric antigen receptor (CAR) or a T cell receptor (TCR).
[10] The immune cell according to [1], wherein the cell surface molecule is a chimeric antigen receptor (CAR) and a T cell receptor (TCR).
[11] The immune cell according to [1], wherein the iPS cell is an iPS cell obtained by reprogramming a peripheral blood mononuclear cell from which a B cell and a T cell have been removed, or a T cell.
[12] The immune cell according to [1], wherein the tumor-related antigen is selected from the group consisting of WT1, GPC3, BCMA, XAGE1, MUC1, MUCSA1, MUC6, EGFRvIII, HER-2/neu, MAGE-A1, MAGE-A3, telomerase, PRAME, SSX2/4, PSCA, CTLA-4, gp100, GD2, GD3, fucosyl GM1, GM3, sLe(a), glycolipid F77, mesothelin, PD-L1, trp1, trp2, CD19, CD20, CD22, ROR1, CD33, c-Met, p53 without gene mutation, p53 with gene mutation, a p53 mutant, NY-ESO-1, PSMA, ETV6-AML, CEA, PSA, AFP, hTERT, EpCAM, ALK, an androgen receptor, EphA2, CYP1B1, OY-TES-1, MAD-CT-2, Melan A/MART 1, survivin, Ras, a Ras mutant, EGR, bcr-ab1, XBP-1, a neo-antigen resulting from gene mutation, a neo-antigen resulting from abnormal splicing, HBV, HBs, HPV, EBV, LMP1, EBV, LMP2, EBNA, HPV-E1, HPV-E2, HPV-E6, HPV-E7, HTLV-1 Tax, and HBZ.
[13] A method for producing the immune cell according to [3], comprising:
   (1) a step of introducing a nucleic acid encoding the cell surface molecule into an iPS cell, or a hematopoietic stem cell, an immature immune cell, or a mature immune cell induced to differentiate from an iPS cell;
   (2) a step of including the following step (2-1), (2-2), or (2-3) of:
      (2-1) differentiating the iPS cell having the nucleic acid introduced thereinto obtained in the step (1) into a hematopoietic stem cell, an immature immune cell, or a mature immune cell;
      (2-2) differentiating, into an immature immune cell or a mature immune cell, the hematopoietic stem cell induced to differentiate from the iPS cell, having the nucleic acid introduced thereinto, obtained in the step (1); or
      (2-3) differentiating, into a mature immune cell, the immature immune cell induced to differentiate from the iPS cell, having the nucleic acid introduced thereinto, obtained in the step (1); and
   (3) a step of introducing a nucleic acid encoding IL-15 into the mature immune cell having the nucleic acid introduced thereinto obtained in the step (1), or the hematopoietic stem cell, the immature immune cell, or the mature immune cell obtained in the step (2-1), (2-2), or (2-3).
[14] A method for producing the immune cell according to [4], comprising:
   (1) a step of introducing a nucleic acid encoding the cell surface molecule into an iPS cell, or a hematopoietic stem cell, an immature immune cell, or a mature immune cell induced to differentiate from an iPS cell;
   (2) a step of including the following step (2-1), (2-2), or (2-3) of:
      (2-1) differentiating the iPS cell having the nucleic acid introduced thereinto obtained in the step (1) into a hematopoietic stem cell, an immature immune cell, or a mature immune cell;
      (2-2) differentiating, into an immature immune cell or a mature immune cell, the hematopoietic stem cell induced to differentiate from the iPS cell, having the nucleic acid introduced thereinto, obtained in the step (1); or
      (2-3) differentiating, into a mature immune cell, the immature immune cell induced to differentiate from the iPS cell, having the nucleic acid introduced thereinto, obtained in the step (1); and
   (3) a step of introducing a nucleic acid encoding IL-15, and a nucleic acid encoding IL-12, IL-18, or IL-21 into the mature immune cell having the nucleic acid introduced thereinto obtained in the step (1), or the hematopoietic stem cell, the immature immune cell, or the mature immune cell obtained in the step (2-1), (2-2), or (2-3).
[15] A method for producing the immune cell according to [5], comprising:
   (1) a step of introducing an expression vector containing a nucleic acid encoding the cell surface molecule into an iPS cell, or a hematopoietic stem cell, an immature immune cell, or a mature immune cell induced to differentiate from an iPS cell;
   (2) a step of including the following step (2-1), (2-2), or (2-3) of:
      (2-1) differentiating the iPS cell having the expression vector introduced thereinto obtained in the step (1) into a hematopoietic stem cell, an immature immune cell, or a mature immune cell;
      (2-2) differentiating, into an immature immune cell or a mature immune cell, the hematopoietic stem cell induced to differentiate from the iPS cell, having the expression vector introduced thereinto, obtained in the step (1); or
      (2-3) differentiating, into a mature immune cell, the immature immune cell induced to differentiate from the iPS cell, having the expression vector introduced thereinto, obtained in the step (1); and
   (3) a step of introducing an expression vector containing a nucleic acid encoding IL-15 into the mature immune cell having the expression vector introduced thereinto obtained in the step (1), or the hematopoietic stem cell, the immature immune cell, or the mature immune cell obtained in the step (2-1), (2-2), or (2-3).
[16] A method for producing the immune cell according to [6], comprising:
   (1) a step of introducing an expression vector containing a nucleic acid encoding the cell surface molecule into an iPS cell, or a hematopoietic stem cell, an immature immune cell, or a mature immune cell induced to differentiate from an iPS cell;
   (2) a step of including the following step (2-1), (2-2), or (2-3) of:
      (2-1) differentiating the iPS cell having the expression vector introduced thereinto obtained in the step (1) into a hematopoietic stem cell, an immature immune cell, or a mature immune cell;
      (2-2) differentiating, into an immature immune cell or a mature immune cell, the hematopoietic stem cell induced to differentiate from the iPS cell, having the expression vector introduced thereinto, obtained in the step (1); or
      (2-3) differentiating, into a mature immune cell, the immature immune cell induced to differentiate from the iPS cell, having the expression vector introduced thereinto, obtained in the step (1); and
   (3) a step of introducing an expression vector containing a nucleic acid encoding IL-15, and a nucleic acid encoding IL-12, IL-18, or IL-21 into the mature immune cell having the expression vector introduced thereinto obtained in the step (1), or the hematopoietic stem cell, the immature immune cell, or the mature immune cell obtained in the step (2-1), (2-2), or (2-3).
[17] A pharmaceutical comprising the immune cell according to any one of [1] to [12].
[18] The pharmaceutical according to [17], for use in prevention or treatment of cancer.
[19] A cytotoxic agent for a cell expressing a tumor-related antigen, comprising the immune cell according to any one of [1] to [12].
[20] A method for preventing or treating cancer in a mammal, comprising administering, to the mammal, an effective amount of the immune cell according to any one of [1] to [12].
[21] A method for preventing or treating cancer in a mammal, comprising administering, to the mammal, an effective amount of the pharmaceutical according to [17].
[22] A method for preventing or treating cancer in a mammal, comprising administering, to the mammal, an effective amount of the cytotoxic agent according to [19].
[23] An agent for preventing or treating cancer in a mammal, comprising the immune cell according to any one of [1] to [12].
[24] The immune cell according to any one of [1] to [12], for use in prevention or treatment of cancer.
[25] The immune cell according to any one of [1] to [12], for producing an agent for preventing or treating cancer.

### Advantageous Effects of Invention

An immune cell induced from an iPS cell, wherein the immune cell expresses a cell surface molecule reactive to a tumor-related antigen and IL-15, and the immune cell further expressing IL-12, IL-18, or IL-21, according to the present invention are improved and retained in cytotoxic activity against a target cell expressing the tumor-related antigen, cell proliferative capacity, and anti-apoptotic ability. Besides, the immune cells of the present invention have an enhanced ability to migrate into solid tumor, and an enhanced long-term viability in solid tumor, and therefore can inhibit growth of the solid tumor and improve the survival rate of an individual. A pharmaceutical or a cytotoxic agent for a cell expressing a tumor-related antigen, containing the immune cell of the present invention are useful for prevention or treatment of cancer.

### Brief Description of Drawings

[Figure 1] Figure 1 is a schematic diagram of an expression vector for introducing a GPC3-reactive CAR gene (GPC3-CAR) into an iPS cell.
[Figure 2A] Figure 2A illustrates results of flow cytometry analysis of surface markers of T cells differentiated from iPS cells having GPC3-CAR introduced thereinto (iPS-CAR-T cells) and fresh PBMC (peripheral blood mononuclear cells).
[Figure 2B] Figure 2B illustrates results of flow cytometry analysis of CAR expressed in iPS-CAR-T cells.
[Figure 3] Figure 3 is a schematic diagram of an expression vector for introducing a gene of IL-15, or IL-15 and IL-12, IL-18 or IL-21 into a GPC3-reactive iPS-CAR-T cell.
[Figure 4] Figure 4 illustrates results of measuring, by ELISA method, amounts of IL-15, IL-12, IL-18, and IL-21 produced by GPC3-reactive iPS-CAR-T cells having cytokine gene introduced thereinto.
[Figure 5] Figure 5 illustrates results of examining the effect of cytokine gene transfer on cell proliferative capacity of the GPC3-reactive iPS-CAR-T cells by PHA and PBMC stimulation.
[Figure 6] Figure 6 illustrates cytotoxic activity of cytokine-expressing GPC3-reactive iPS-CAR-T cells.
[Figure 7A] Figure 7A illustrates results of measuring over time of cytotoxicity to the skHep-GPC3 cells obtained by co-culturing cytokine-expressing GPC3-reactive iPS-CAR-T cells with SK-HEP-1 cell having GPC3 gene introduced thereinto.
[Figure 7B] Figure 7B illustrates results of measuring over time of proliferation of the skHep-GPC3 cells obtained by co-culturing cytokine-expressing GPC3-reactive iPS-CAR-T cells with SK-HEP-1 cell having GPC3 gene introduced thereinto.
[Figure 7C] Figure 7C illustrates results of measuring over time of proliferation of the iPS-CAR-T cells obtained by co-culturing cytokine-expressing GPC3-reactive iPS-CAR-T cells with SK-HEP-1 cell having GPC3 gene introduced thereinto.
[Figure 8] Figure 8 illustrates results of measuring apoptotic cells by staining the cytokine-expressing GPC3-reactive iPS-CAR-T cells with Annexin V and PI (Propidium Iodide) in the presence or absence of the SK-HEP-1 cell having GPC3 gene introduced thereinto.
[Figure 9A] Figure 9A illustrates results of evaluating migration ability of the cytokine-expressing GPC3-reactive iPS-CAR-T cells by Transwell (R) assay using culture supernatants of JHH-7 cell and skHep-GPC3 cell.
[Figure 9B] Figure 9B illustrates results of flow cytometry analysis of expression of CXCR3 and CCR5 in the cytokine-expressing GPC3-reactive iPS-CAR-T cells.
[Figure 9C] Figure 9C illustrates results of evaluating the migration ability of the cytokine-expressing GPC3-reactive iPS-CAR-T cells to CXCL9, CXCL10, and CXCL11 by Transwell (R) assay.
[Figure 10A] Figure 10A illustrates influence of an anti-human CXCR3 antibody on migration, to the periphery of mouse tumor, of IL-15- and IL-21-expressing GPC3-reactive iPS-CAR-T cells, as measured by *in vivo* imaging using luciferase luminescence.
[Figure 10B] Figure 10B illustrates analysis results of influence of an anti-human CXCR3 antibody on infiltration, into mouse tumor, of IL-15- and IL-21-expressing GPC3-reactive iPS-CAR-T cells.
[Figure 10C] Figure 10C illustrates analysis results of influence of an anti-human CXCR3 antibody on proliferation ability of IL-15- and IL-21-expressing GPC3-reactive iPS-CAR-T cells.
[Figure 10D] Figure 10D illustrates results of flow cytometry analysis of CXCR3 expression in GPC3-reactive iPS-CAR-T cells having IL-15 and IL-21 genes introduced thereinto, infiltrated into mouse tumor.
[Figure 11A] Figure 11A illustrates results of measuring CXCR3 expression in cytokine-expressing GPC3-reactive iPS-CAR-T cells by quantitative PCR.
[Figure 11B] Figure 11B illustrates results of Western blotting analysis of expression of phosphorylated STAT1, STAT3 and STATS in the cytokine-expressing GPC3-reactive iPS-CAR-T cells.
[Figure 11C] Figure 11C illustrates analysis results of relative expression levels of phosphorylated STAT1, STAT3, and STATS in the cytokine-expressing GPC3-reactive iPS-CAR-T cells.
[Figure 11D] Figure 11D illustrates a region amplified by ChIP-qPCR in a CXCR3 promoter region.
[Figure 11E] Figure 11E illustrates results of verifying involvement of STAT1, STAT3, and STATS in CXCR3 transcription by ChIP-qPCR.
[Figure 12] Figure 12 illustrates results of observing over time by *in vivo* imaging using luciferase luminescence of *in vivo* kinetics of the iPS-CAR-T cells obtained by intravenous injection of the GPC3-reactive iPS-CAR-T cells having cytokine gene introduced thereinto, to NSG mice in which tumor has been formed by human liver cancer-derived cell line SK-HEP-1 having GPC3 gene introduced thereinto.
[Figure 13A] Figure 13A illustrates results of measuring over time of tumor volumes, obtained after administration of GPC3-reactive iPS-CAR-T cells having cytokine gene introduced thereinto, in NSG mice subcutaneously inoculated with human liver cancer cell line JHH-7.
[Figure 13B] Figure 13B illustrates survival rates over time of the mice illustrated in Figure 13A.
[Figure 14A] Figure 14A illustrates results of measuring over time of tumor volumes, obtained after administration of GPC3-reactive iPS-CAR-T cells having cytokine gene introduced thereinto, in NSG mice subcutaneously inoculated with human liver cancer-derived cell line SK-HEP-1 having GPC3 gene introduced thereinto.
[Figure 14B] Figure 14B is illustrates survival rates over time of the mice illustrated in Figure 14A.
[Figure 15] Figure 15 illustrates comparison of the amounts, present in a tumor, of the GPC3-reactive iPS-CAR-T cells having cytokine gene introduced thereinto.
[Figure 16] Figure 16 illustrates results of single cell RNA-sequence analysis plotted using UMAP.
[Figure 17] Figure 17 illustrates clusters plotted on UMAP, and distributions of the iPS-CAR-T cells having cytokine gene introduced thereinto.
[Figure 18] Figure 18 illustrates analysis results of a gene group of Cluster 1 shown in Figure 16.
[Figure 19] Figure 19 illustrates analysis results of a gene group of Cluster 4 shown in Figure 16.
[Figure 20] Figure 20 illustrates analysis results of gene groups of Clusters 1 and 4, which are composed of IL-15- and IL-21-expressing iPS-CAR-T cells, as shown in Figure 17.
[Figure 21] Figure 21 illustrates that the IL-15- and IL-21-expressing iPS-CAR-T cells have a young memory phenotype.
[Figure 22] Figure 22 illustrates tumor infiltration ability of iPS cell-derived TCR-T cells enhanced by IL-15 and IL-21.
[Figure 23] Figure 23 illustrates tumor proliferation inhibitory effect of iPS cell-derived TCR-T cells owing to IL-15 and IL-21.
[Figure 24] Figure 24 illustrates tumor proliferation inhibitory effect of iPS cell-derived TCR-T cells owing to IL-15 and IL-21.

### Description of Embodiments

### [Immune Cell Induced from iPS Cell]

An immune cell induced from an iPS cell of the present invention is not especially limited as long as it expresses a cell surface molecule reactive to a tumor-related antigen, and IL-15. In one embodiment of the present invention, the immune cell induced from an iPS cell expresses IL-12, IL-18, or IL-21 in addition to the cell surface molecule reactive to a tumor-related antigen, and IL-15. In one embodiment of the present invention, the immune cell induced from an iPS cell may co-express all of IL-12, IL-18, and IL-21, or may express two cytokines selected from the group consisting of IL-12, IL-18, and IL-21. Besides, the immune cell induced from an iPS cell of the present invention may express any other cytokines and chemokines controlling cell functions and immune functions such as cytotoxic activity, cell proliferation, and cell migration, and receptors of these.

In the present invention, the term "tumor-related antigen" refers to an antigen that is specifically or non-specifically expressed in tumors, and examples thereof include an antigen derived from a protein overexpressed in a tumor cell, and a mutant thereof, a tumor virus-derived antigen, a certain type of differentiation antigen, and a novel tumor-related antigen (neo-antigen) resulting from gene mutation, or abnormal splicing. When the antigen is a protein antigen, it may be a peptide (peptide fragment) fragmented therefrom. Herein, the term "tumor-related antigen" is used in the same meaning as a cancer antigen. Examples of the antigen that is specifically or non-specifically expressed in tumors include, but are not limited to, WT1, GPC3, BCMA, XAGE1, MUC1, MUC5A1, MUC6, EGFRvIII, HER-2/neu, MAGE-A1, MAGE-A3, telomerase, PRAME, SSX2/4, PSCA, CTLA-4, gp100, GD2, GD3, fucosyl GM1, GM3, sLe(a), glycolipid F77, mesothelin, PD-L1, trp1, trp2, CD19, CD20, CD22, ROR1, CD33, c-Met, p53 without gene mutation, p53 with gene mutation, a p53 mutant, NY-ESO-1, PSMA, ETV6-AML, CEA, PSA, AFP, hTERT, EpCAM, ALK, an androgen receptor, EphA2, CYP1B1, OY-TES-1, MAD-CT-2, Melan A/MART1, survivin, Ras, a Ras mutant, EGR, bcr-ab1, XBP-1, a neo-antigen resulting from gene mutation, and neo-antigen resulting from abnormal splicing. Examples of the viral antigen include, but are not limited to, HBV, HBs, HPV, EBV, LMP1, EBV, LMP2, EBNA, HPV-E1, HPV-E2, HPV-E6, HPV-E7, HTLV-1 Tax, and HBZ.

In one embodiment of the present invention, the tumor-related antigen may be selected from the group consisting of GPC3, WT1, BCMA, XAGE1, LMP2, NY-ESO-1, an EB viral antigen, a neo-antigen, and peptide fragments thereof.

In the present invention, the term "cell surface molecule reactive to a tumor-related antigen" refers to a cell surface molecule specifically binding to a tumor-related antigen on an antigen presenting cell such as a cancer cell. In one embodiment of the present invention, the cell surface molecule is a T cell receptor (TCR) and a chimeric antigen receptor (CAR).

The TCR may be a hetero dimer consisting of an α chain and a β chain, or a hetero dimer consisting of a γ chain and a δ chain. The phrase TCR "being reactive to a tumor-related antigen" means that a reaction is caused when an immune cell expressing the TCR selectively binds, through the TCR, to an epitope peptide derived from a tumor-related antigen presented on major histocompatibility complex (MHC) class I or class II on an antigen presenting cell, and refers to that the reaction is not caused in the immune cell when the immune cell binds to another one except for the epitope peptide. Examples of the reaction of the immune cell caused by the bond through the TCR to the epitope peptide derived from a tumor-related antigen presented on the MHC class I or class II include cytotoxicity, production of IFN-y and granzyme, expression of a T cell activation marker, and activation of a transcription factor such as NF-AT.

The CAR is a fusion protein of an antigen recognition site of an antibody specifically recognizing a tumor-related antigen, and an intracellular domain of a T cell activation molecule. The antigen recognition site of the antibody and the intracellular domain of the T cell activation molecule may be bound via a spacer and/or a cell transmembrane domain. An example of the antigen recognition site of the antibody that is an extracellular region of a CAR molecule may include a single chain antibody in which a light chain and a heavy chain of a variable region of a monoclonal antibody against the tumor-related antigen are bound in series. As the intracellular domain of the T cell activation molecule in the CAR molecule, a CD3ζ molecule is used, and for sufficiently activating the immune cell, a costimulatory molecule may be incorporated into the intracellular domain. Examples of the costimulatory molecule include CD27, CD28, 4-IBB, OX40, and ICOS. Two or more of these costimulatory molecules may be incorporated in combination into the intracellular domain. The phrase CAR "being reactive to a tumor-related antigen" refers to that the immune cell expressing the CAR specifically binds, via an antigen recognition site of the CAR molecule, to a tumor-related antigen expressed on an antigen presenting cell, and thus, an activation signal is sent to the immune cell via a CD3ζ molecule in the intracellular domain of the CAR molecule. An example of the reaction of the immune cell caused through the CAR includes apoptosis induction of an antigen presenting cell through release of a cytotoxic protein such as perforin or granzyme. The immune cell expressing the CAR can recognize an antigen expressed on an antigen presenting cell surface without being constrained by a human leukocyte antigen (HLA).

### [Production of iPS Cell]

In the present invention, an iPS cell is produced by reprogramming a somatic cell of a mammal. Examples of the mammal include a human, a monkey, a pig, a dog, a cat, a rat, and a mouse, and a human is preferred. The somatic cell is not especially limited, but a cell isolated from peripheral blood is suitably used. In one embodiment of the present invention, the somatic cell is a peripheral blood mononuclear cell from which a B cell and a T cell have been removed, or a T cell. The peripheral blood mononuclear cell from which a B cell and a T cell have been removed may be obtained by isolating a mononuclear cell from whole blood using a mononuclear cell separation solution, and then removing a B cell and a T cell using surface antigens expressed on the B cell and the T cell. An example of the mononuclear cell separation solution includes Lymphoprep (R). For removing a B cell and a T cell from a mononuclear cell, antibodies against CD19, CD20, CD22, or a B cell receptor that is a surface antigen of a B cell, and CD3, CD4, or CD8 that is a surface antigen of a T cell may be used, and for example, flow cytometry, or magnetic beads such as MACS (R) beads may be used.

A preferable example of a T cell source includes, but is not limited to, peripheral blood because of low invasiveness. Other examples of the source include a cancer tissue, a tumor tissue or another tissue, umbilical cord blood, lymph, a tissue fluid (an inter-tissue fluid, an intracellular fluid, and an interstitial fluid), a body cavity fluid (an ascites fluid, a pleural fluid, a pericardial fluid, a cerebrospinal fluid, a synovial fluid, and aqueous humor), nasal discharge, and an exudate in the urinary thoracic cavity, the abdominal cavity, the cranial cavity, or the spinal canal (pleural fluid, ascites fluid, or the like). Examples of the cancer tissue or tumor tissue include tissues derived from ovarian cancer, hepatoblastoma, hepatocellular carcinoma, stomach cancer, esophageal cancer, pancreatic cancer, renal cell cancer, breast cancer, malignant melanoma, non-small cell lung cancer, cervical cancer, glioblastoma, prostate cancer, neuroblastoma, chronic lymphocytic leukemia, papillary thyroid cancer, colorectal cancer, head and neck cancer, brain tumor, multiple myeloma, and B-cell non-Hodgkin's lymphoma. In one embodiment of the present invention, the cancer tissue or tumor tissue is hepatocellular carcinoma or hepatoblastoma.

A method for producing an iPS cell is known in the art (see, for example, Takahashi K., Yamanaka S., Cell. 2006; 126: 663-676, Takahashi K. et al., Cell. 2007; 131: 861-872, and Nakagawa M. et al., Nat Biotechnol. 2008; 26: 101-106). In one embodiment of the present invention, an iPS cell can be induced by introducing a cell reprogramming factor into a peripheral blood mononuclear cell from which a cell and a T cell have been removed, or a T cell. Examples of the cell reprogramming factor include genes or gene products such as Oct3/4, Sox2, Sox1, Sox3, Sox15, Sox17, klf4, klf2, c-Myc, N-Myc, L-Myc, Nanog, Lin28, Fbx15, ERas, ECAT15-2, Tcl1, β-catenin, Lin28b, Sall1, Sall4, Esrrb, Nr5a2, Tbx3, and Glis1. One of these cell reprogramming factors may be singly used, or a combination thereof may be used. Among these cell reprogramming factors, Oct3/4, Sox2, Klf4, and c-Myc (what is called Yamanaka four factors) may be introduced into the peripheral blood mononuclear cell or T cell from the viewpoint that an iPS cell can be thus efficiently established.

A method for introducing the cell reprogramming factor into the peripheral blood mononuclear cell or T cell is not especially limited, and any method known in the art may be employed. For example, in introducing a gene encoding the cell reprogramming factor into the peripheral blood mononuclear cell or T cell, a gene (for example, a cDNA) encoding the cell reprogramming factor may be inserted into an expression vector containing a promoter functioning in the peripheral blood mononuclear cell or T cell, and the resultant expression vector may be introduced into the peripheral blood mononuclear cell or T cell by infection, a lipofection method, a liposome method, a calcium phosphate coprecipitation method, a DEAE dextran method, a microinjection method, or an electroporation method. When the cell reprogramming factor is in the form of a protein, and the protein is to be introduced into the peripheral blood mononuclear cell or T cell, a method using a protein transfection reagent, a method using a protein transduction domain fusion protein, an electroporation method, or a microinjection method may be employed. When the cell reprogramming factor is in the form of a messenger RNA (mRNA), and the mRNA is to be introduced into the peripheral blood mononuclear cell or T cell, a method using an mRNA transfection reagent, or a method in which the mRNA is added to a culture fluid may be employed.

Examples of the expression vector used for gene transfer by infection include viral vectors such as a lentivirus, a retrovirus, an adenovirus, an adeno-associated virus, a herpes virus, and a Sendai virus, and an animal cell expression plasmid. From the viewpoints that insertion mutation is difficult to occur, gene transfer efficiency is high, and the copy number of the gene to be introduced is also large, a Sendai virus may be used for introducing a gene encoding the cell reprogramming factor into the peripheral blood mononuclear cell or T cell.

Examples of the promoter used in the expression vector, which is used in introducing a gene encoding the cell reprogramming factor into the peripheral blood mononuclear cell or T cell, include an SRα promoter, an SV40 promoter, an LTR promoter, a CMV promoter, an RSV promoter, an HSV-TK promoter, and a ubiquitin promoter. These promoters may be capable of controlling expression of a gene inserted downstream of the promoter depending on the presence or absence of a drug such as tetracycline. The expression vector may contain, in addition to the promoter, an enhancer, a poly A addition signal, a selection marker gene (for example, neomycin resistance gene), SV40 replication origin, and the like.

### [Introduction, into iPS cell, of Cell Surface Molecule Reactive to Tumor-related Antigen]

In the present invention, into the iPS cell obtained as described above, a nucleic acid encoding a cell surface molecule reactive to a tumor-related antigen is introduced from outside the cell. The nucleic acid encoding a cell surface molecule may be introduced into a hematopoietic stem cell, an immature immune cell, or a mature immune cell induced to differentiate from an iPS cell. The nucleic acid encoding a cell surface molecule is preferably a human-derived nucleic acid. Examples of the nucleic acid encoding a cell surface molecule reactive to a tumor-related antigen may include a nucleic acid encoding a TCR, and a nucleic acid encoding a CAR, and the nucleic acid may be a naturally-derived nucleic acid or an artificially synthesized nucleic acid. Sequence information of these nucleic acids is available from known literature and databases such as NCBI (http://www.ncbi.nlm.nih.gov/guide/).

When the cell surface molecule is a TCR, cDNAs encoding TCR α chain and β chain, or cDNAs encoding TCR γ chain and δ chain may be prepared to be incorporated into an expression vector. These cDNAs may be incorporated into a viral vector or non-viral vector (transposon vector) using, for example, Gibson assembly system. Specifically, for introducing a TCR, a gene in which cDNAs encoding TCR α chain and TCR β chain are linked via a T2A sequence may be bound downstream of a ubiquitin promoter, and further downstream of this, a marker gene such as an EGFR from which a ligand binding site and an intracellular domain have been removed (EGFRt, truncated EGFR), or CD19 deficient in the intracellular domain may be linked to an IRES (internal ribosome entry site) sequence. The resultant construct may be incorporated into a viral vector or non-viral vector. A cDNA encoding TCR α chain and a cDNA encoding TCR β chain may be incorporated respectively into different expression vectors.

In one embodiment of the present invention, the cDNAs respectively encoding the TCR α chain and β chain may be prepared with respect to a single cell by using, as a supply source, a T cell population that is reactive to a tumor-related antigen, and has genetic diversity as a whole. T cells obtained from a subject may be cultured together with a target tumor-related antigen, and then, a single cell may be isolated from a T cell population responsive to the used tumor-related antigen with a cell sorter or the like by using an activation marker. An example of the activation marker includes cell surface CD137. An example of a known method for isolating a human T cell includes flow cytometry using an antibody against a T cell surface marker, such as CD3 or CD137, and a cell sorter. The thus obtained single T cell is subjected to gene cloning by PCR, and thus, the cDNAs respectively encoding the TCR α chain and β chain can be amplified.

For obtaining a single T cell, a CD8 single positive T cell that is obtained from peripheral blood or the like, and is specific to a tumor-related antigen may be subjected to single cell sorting with a cell sorter through binding to an MHC Dextramer (R) forming a complex with an antigen peptide. The MHC dextramer is a compound composed of a dextran polymer skeleton containing MHC bound to a fluorescent dye molecule. An MHC tetramer may be used instead of the MHC dextramer. The MHC tetramer is obtained by tetramerizing, with biotin and avidin, a complex of an antigen peptide and an MHC molecule. In another aspect, a CD8 single positive T cell that is obtained from peripheral blood or the like, and is specific to a tumor-related antigen may be expansion-cultured in the presence of the antigen, and then a CD3/CD137 double positive cell may be subjected to single cell sorting using a cell sorter. In another aspect, from CD3/CD137 double positive cells, a cell population bound to an MHC dextramer forming a complex with an antigen peptide may be subjected to single cell sorting using a cell sorter. An RNA may be extracted from the thus obtained single cell, and a cDNA obtained by reverse transcription reaction may be used for isolating a TCR gene pair (TCR α chain gene and TCR β chain gene) by PCR. The thus isolated TCR gene pair may be subjected to sequence analysis so as to analyze the type of tumor antigen-reactive T cell (TCR repertoire) and the frequency of appearance thereof.

When the cell surface molecule is a CAR, a cDNA encoding the CAR may be prepared to be incorporated into an expression vector. For example, a CAR construct can be prepared as follows: A total RNA is extracted from the lymph node of an animal immunized with a desired tumor-related antigen, and the resultant is used for synthesizing a cDNA; a light chain and a heavy chain of a variable region of a monoclonal antibody against the tumor-related antigen are separately amplified, the amplified chains are linked via a flexible linker, and the resultant is amplified by assembly PCR; a sequence encoding the fused light chain and heavy chain is bound, through a sequence encoding a cell transmembrane domain of a CAR molecule, to a sequence encoding an intracellular domain of the CAR molecule. To this CAR construct, an EGFR in which a ligand binding site and an intracellular domain have been removed (EGFRt) may be linked via a T2A sequence for confirming expression of the introduced gene. The flexible linker used for linking a light chain and a heavy chain of a variable region of a monoclonal antibody is a linker peptide having about 5 to 20 amino acid residues, and is known in the art (see, for example, Ueda T. et al., Cancer Sci. 2020; 111: 1478-1490, Tomomi Kawasaki et al., SCEJ 72nd Annual Meeting (Kyoto, 2007) H209).

An antigen-binding domain incorporated into the CAR may be prepared by phage display without immunizing an animal with an antigen. For example, from a phage display antibody library expressing Fab regions of a large number of human antibodies, an antibody specifically binding to a desired tumor-related antigen may be screened.

A gene encoding one or more of the cell surface molecules may be introduced into the iPS cell. For example, a TCR or a CAR reactive to a tumor-related antigen may be singly introduced, or the TCR and the CAR may be simultaneously introduced. Expression vectors used in simultaneously introducing the TCR and the CAR may be the same or different. When the TCR and the CAR are simultaneously introduced, the TCR and the CAR may be reactive to the same tumor-related antigen, or may be reactive to different tumor-related antigens. The gene encoding the cell surface molecule may be introduced into a hematopoietic stem cell differentiated from an iPS cell, or an immature immune cell or mature immune cell differentiated from the hematopoietic stem cell. Here, the term "immature immune cell" encompasses cells at all differentiation stages in differentiation from a hematopoietic stem cell to a mature immune cell, and is used in the meaning including, for example, a precursor immune cell and an immature immune cell.

As the expression vector, a viral vector and a non-viral vector can be used. Examples of the viral vector include a lentivirus, a retrovirus, an adenovirus, an adeno-associated virus, a herpes virus, and a Sendai virus, as well as an animal cell expression plasmid. A lentivirus or a retrovirus may be suitably used. In performing infection with a retrovirus or a lentivirus, a spin infection method or the like may be employed. An example of the non-viral vector may include a piggyBac (R) vector that is a transposon vector. When a TCR endogenous in an iPS cell is to be replaced with a TCR or a CAR introduced from outside the cell, genome editing technology may be employed. Examples of such genome editing technology include CRISPR/Cas9 method, CRISPR/MAD method, and CRISPR/CAS3 method. Examples of a method for introducing a gene of a non-viral vector, or a method for introducing a guide RNA and a donor DNA for genome editing include a lipofection method, a liposome method, a calcium phosphate coprecipitation method, a DEAE dextran method, a microinjection method, and an electroporation method. The gene transfer of a TCR and/or a CAR may be performed on a gene locus of the TCR, or another gene locus (for example, β2-microglobulin gene locus). When modification or disruption of existing genes including TCR by the introduced gene is unwanted, the gene transfer may be performed on a safe harbor gene locus. An example of the safe harbor includes AAVS1 (Adeno-associated virus integration site 1) region in the human genome. Examples of a method for introducing a gene targeting the safe harbor include CRISPR/Cas9 method and TALEN method.

iPS cells having a cell surface molecule reactive to a tumor-related antigen introduced thereinto consist of a large number of iPS cell clones. Therefore, from the aggregation of the iPS cell clones, iPS cell clones that have been confirmed in the gene transfer of the cell surface molecule, and retain prescribed properties may be selected. An example of a method for selecting iPS cell clones includes a colony pickup method. The colony pickup method is not especially limited, and a method using a Pipetman under a microscope, a limiting dilution method, a method using a fully automatic colony picker, or the like may be employed. The thus obtained single iPS cell clone may be cryopreserved after being expansion-cultured. A method for cryopreserving a cell is known to those skilled in the art. For example, a cultured iPS cell clone is collected, and washed with a buffer or culture fluid, the number of cells is counted, the resultant is concentrated by centrifugation or the like, and suspended in freezing medium (for example, a culture fluid containing 10% DMSO), and then the resultant may be cryopreserved at a low temperature. In the cryopreservation, the preservation temperature is not especially limited as long as it is suitable for preserving cells. The temperature is, for example, -20°C, -80°C, or -120 to -196°C, and is preferably -150°C or lower.

The culture fluid used in the culture of the iPS cell is not especially limited, and can be prepared by using a culture fluid used in culture of animal cells as a basal culture fluid, and adding thereto cytokines for retaining the undifferentiability of an iPS cell. Examples of the basal culture fluid include Iscove's Modified Dulbecco's Medium (IMDM) culture fluid, Medium199 culture fluid, Eagle's Minimum Essential Medium (EMEM) culture fluid, αMEM culture fluid, Dulbecco's modified Eagle's Medium (DMEM) culture fluid, Ham's F12 culture fluid, RPMI1640 culture fluid, Fischer's culture fluid, Neurobasal Medium (Life Technologies Corporation), StemFit (R) AK03N (Ajinomoto Healthy Supply Co., Inc.), and mixtures of these culture fluids. A serum may be added to the culture fluid, or the culture fluid may be serum-free. A preferable example of the cytokines includes bFGF, and the concentration thereof in the culture fluid is, for example, 1 to 100 µg/mL.

### [Differentiation into Immune Cell from iPS Cell Having Cell Surface Molecule Reactive to Tumor-related Antigen Introduced thereinto]

In the present invention, an immune cell to be differentiated from the iPS cell is not especially limited as long as it has immune response ability, and has ability to kill a cell expressing the target tumor-related antigen, and examples thereof include lymphocytic cells such as a T cell, a B cell, a natural killer (NK) cell, and an NKT cell. In one embodiment of the present invention, the immune cell is a T cell or an NK cell. In one embodiment of the present invention, the T cell is a mature T cell, and a CD8 single positive cytotoxic T cell.

In one embodiment of the present invention, an iPS cell clone having cell surface molecule reactive to a tumor-related antigen introduced thereinto is differentiated into a mature T cell, that is, a CD8 single positive T cell, via a hematopoietic stem cell and an immature T cell. The hematopoietic stem cell is a cell capable of differentiating into a hematopoietic cell such as a lymphocyte, an eosinophil, a neutrophil, a basophil, an erythrocyte, or a megakaryocyte, and is recognized by being positive for both CD34 and CD43, that is, surface antigens. It is noted that a hematopoietic stem cell and hematopoietic progenitor cell (HPC) are not distinguished from each other, and refer to the same cell unless otherwise stated. The immature T cell refers to a cell at any of all differentiation stages in differentiation from a hematopoietic stem cell to a mature T cell, and encompasses, for example, a precursor T cell and an immature T cell. Accordingly, the immature T cell is a T cell at each stage from a stage of a T cell at which both TCR α chain and β chain are not expressed up to a stage at which TCR α chain and β chain are expressed to be a CD4/CD8 double positive cell. In one embodiment of the present invention, a mature immune cell induced from an iPS cell is a T cell that has expressed the TCR α chain and β chain, and has differentiated into a CD8 single positive cell through a CD4/CD8 double positive cell, and is preferably CD8 α chain/β chain double positive.

The hematopoietic stem cell may be produced by culturing an iPS cell in a culture fluid supplemented with vitamin C. Vitamin C refers to L-ascorbic acid and derivatives thereof. Examples of the L-ascorbic acid derivatives include phosphoric acid vitamin C, ascorbic acid glucoside, ascorbyl ethyl, vitamin C ester, ascorbyl tetrahexyldecanoate, ascorbyl stearate, and ascorbic 2-phosphate 6-palmitate. The vitamin C is contained, for example, in a concentration of 5 to 500 µg/mL in the culture fluid.

A culture fluid used for producing the hematopoietic stem cell is not especially limited, and can be prepared by using a culture fluid used in culture of animal cells as a basal culture fluid, and adding vitamin C and the like thereto. Examples of the basal culture fluid include Iscove's Modified Dulbecco's Medium (IMDM) culture fluid, Medium199 culture fluid, Eagle's Minimum Essential Medium (EMEM) culture fluid, αMEM culture fluid, Dulbecco's modified Eagle's Medium (DMEM) culture fluid, Ham's F12 culture fluid, RPMI1640 culture fluid, Fischer's culture fluid, Neurobasal Medium (Life Technologies Corporation), StemPro34 (Life Technologies Corporation), and mixtures of these culture fluids. The culture fluid may contain a serum, or may be serum-free. The basal culture fluid may also contain, if necessary, one or more substances selected from, for example, albumin, insulin, transferrin, selenium, fatty acid, trace elements, 2-mercaptoethanol, thiolglycerol, monothiolglycerol, a lipid, an amino acid, L-glutamine, a non-essential amino acid, a vitamin, a growth factor, a low molecular compound, an antibiotics, an antioxidant, pyruvic acid, a buffer, an inorganic salt, and a cytokine.

To the culture fluid used for producing the hematopoietic stem cell, a cytokine selected from the group consisting of BMP4 (bone morphogenetic protein 4), VEGF (vascular endothelial growth factor), bFGF (basic fibroblast growth factor), SCF (stem cell factor), TPO (thrombopoietin), and FLT3L (Flt3 ligand) may be further added. As for concentrations of these in the culture fluid, for example, that of BMP4 is 1 to 100 ng/mL, that of VEGF is 1 to 100 ng/mL, that of bFGF is 1 to 100 ng/mL, that of SCF is 10 to 100 ng/mL, that of TPO is 1 to 100 ng/mL, and that of FLT3L is 1 to 100 ng/mL.

To the culture fluid of a hematopoietic stem cell, a TGFβ inhibitor may be added. The TGFβ inhibitor refers to a low molecular inhibitor that interferes signaling of TGFβ family, examples thereof include SB431542 and SB202190 (see, for example, Lindemann R. K. et al., Mol Cancer. 2003; 2: 20), SB505124 (GlaxoSmithKline), NPC30345, SD093, SD908, and SD208 (Scios), and LY2109761, LY364947, and LY580276 (Lilly Research Laboratories), and the concentration thereof to be added to the culture fluid is preferably 0.5 to 100 µM.

The iPS cell may be co-cultured with a feeder cell such as C3H10T1/2 (see, for example, Takayama N. et al., J Exp Med. 2010; 2817-2830), or a heterologous stromal cell (see, for example, Niwa A. et al., J Cell Physiol. 2009; 221: 367-377).

A method for culturing the iPS cell in producing a hematopoietic stem cell may be adherent culture or suspension culture, but is preferably suspension culture. For example, the iPS cell may be subjected to suspension culture after separating a colony having been cultured to 80% confluent in a used dish, and dissociating the colony into a single cell. Examples of a method for isolating the iPS cell include a method of physical isolation with a cell scraper or the like, and an isolation method using a dissociation solution having protease activity and collagenase activity (such as Accutase (R) or Accumax (R)), or a dissociation solution having collagenase activity.

The suspension culture refers to culturing cells in a non-adherent state to a culture vessel. The suspension culture may be performed by using a culture vessel, although not especially limited, not subjected to an artificial treatment for improving the adhesiveness to a cell (for example, a coating treatment with an extracellular matrix or the like), or a culture vessel subjected to a treatment for artificially inhibiting adhesion (for example, a coating treatment with polyhydroxyethyl methacrylic acid (poly-HEMA) or a nonionic surfactant polyol (such as Pluronic F-127)). The suspension culture is performed preferably with an embryoid body (EB) formed. When a hematopoietic stem cell is obtained through suspension culture of an embryoid body, adherent culture may be performed after dissociating the resultant into a single cell.

The hematopoietic stem cell may be also prepared from a cyst-like structure (also referred to as an iPS-sac) obtained by culturing an iPS cell. Here, the cyst-like structure refers to a three-dimensional sac-like structure having a space inside derived from an iPS cell, and is a structure constituted by an endothelial cell population or the like, and containing a hematopoietic stem cell inside.

A method for culturing the hematopoietic stem cell may be adherent culture or suspension culture, but is preferably adherent culture. In employing the adherent culture, a culture vessel may be coated for use. Examples of a coating agent include Matrigel (see, for example, Niwa A. et al., PLoS One. 2011; 6: e22261), collagen, gelatin, laminin, heparan sulfate proteoglycan, RetroNectin, Fc-DLL4, entactin, and combinations thereof.

A CD4/CD8 double positive T cell is, among T cells, positive for both surface antigens CD4 and CD8 (CD4⁺/CD8⁺), and since a T cell can be recognized by being positive for surface antigens CD3 and CD45, the CD4/CD8 double positive T cell can be identified as a cell positive for CD4, CD8, CD3, and CD45. The CD4/CD8 double positive T cell can be differentiated by induction into a CD4 single positive cell or a CD8 single positive cell.

The CD4/CD8 double positive T cell can be produced by culturing a hematopoietic stem cell in a culture fluid supplemented with a p38 inhibitor and/or SDF-1.

The p38 inhibitor is defined as a substance that inhibits the function of p38 protein (p38 MAP kinase). Examples of the p38 inhibitor include, but are not limited to, a p38 chemical inhibitor, a p38 dominant negative mutant, or a nucleic acid encoding it.

Examples of the p38 chemical inhibitor include, but are not limited to, SB203580 (4-(4-fluorophenyl)-2-(4-methylsulfonylphenyl)-5-(4-pyridyl)-1H-imidazole) and a derivative thereof, SB202190 (4-(4-fluorophenyl)-2-(4-hydroxyphenyl)-5-(4-pyridyl)-1H-imidazole) and a derivative thereof, SB239063 (trans-4-[4-(4-fluorophenyl)-5-(2-methoxy-4-pyrimidinyl)-1H-imidazol-1-yl]cyclohexanol) and a derivative thereof, SB220025 and a derivative thereof, PD169316, RPR200765A, AMG-548, BIRB-796, SCl0-469, SCIO-323, VX-702, or FR167653. These compounds are commercially available, and for example, SB203580, SB202190, SC239063, SB220025, and PD169316 are available from Calbiochem, and SCl0-469 and SCIO-323 are available from Scios Inc. or the like. The p38 inhibitor is added to the culture fluid at a concentration ranging, for example, about 1 µM to about 50 µM.

Examples of the p38 dominant negative mutant include p38T180A obtained by point mutation of threonine at position 180 in DNA binding region of p38 to alanine, and p38Y182F obtained by point mutation of tyrosine at position 182 of p38 in a human or a mouse to phenylalanine.

SDF-1 (Stromal cell-derived factor 1) may be not only SDF-1α or the mature form thereof but also isoforms, such as SDF-1β, SDF-1γ, SDF-1δ, SDF-1ε, or SDF-1ϕ, or the mature forms thereof, or may be a mixture of any of these in an arbitrary ratio. SDF-1α is preferably used. The SDF-1 is added to the culture fluid at a concentration ranging, for example, about 10 ng/mL to about 100 ng/mL.

A culture fluid used for producing the CD4/CD8 double positive T cell is not especially limited, and can be prepared by using a culture fluid used in culture of animal cells as a basal culture fluid, and adding thereto a p38 inhibitor and/or SDF-1, and more preferably vitamin C. Examples of the basal culture fluid include Iscove's Modified Dulbecco's Medium (IMDM) culture fluid, Medium199 culture fluid, Eagle's Minimum Essential Medium (EMEM) culture fluid, αMEM culture fluid, Dulbecco's modified Eagle's Medium (DMEM) culture fluid, Ham's F12 culture fluid, RPMI1640 culture fluid, Fischer's Neurobasal Medium culture fluid (Life Technologies Corporation), and mixtures of these culture fluids. A serum may be added to the culture fluid, or the culture fluid may be serum-free. The basal culture fluid may contain one or more substances selected from, for example, albumin, insulin, transferrin, selenium, fatty acid, trace elements, 2-mercaptoethanol, thiolglycerol, a lipid, an amino acid, L-glutamine, a non-essential amino acid, a vitamin, a growth factor, a low molecular compound, an antibiotics, an antioxidant, pyruvic acid, a buffer, an inorganic salt, and a cytokine.

To the culture fluid used for producing the CD4/CD8 double positive T cell, a cytokine selected from the group consisting of SCF, TPO (thrombopoietin), FLT3L, and IL-7 may be further added. As for the concentrations of these, for example, that of SCF is 10 to 100 ng/mL, that of TPO is 10 to 200 ng/mL, that of FLT3L is 1 to 100 ng/mL, and that of IL-7 is 1 to 100 ng/mL.

The thus obtained CD4/CD8 double positive T cell may be isolated for use. For the isolation, any method known to those skilled in the art may be employed, and examples of the method include a method in which the cell is labeled with an antibody against CD4, CD8, CD3 and/or CD45 to be isolated with a flow cytometer, or a method in which the cell is purified with an affinity column or the like having a desired antigen immobilized therein.

A CD8 single positive T cell, namely, a mature T cell is, among T cells, positive for surface antigen CD8 (CD8⁺ CD4⁻), and is also designated as a cytotoxic T cell. Since a T cell can be recognized by being positive for surface antigens CD3 and CD45, the CD8 single positive T cell can be identified as a cell that is positive for CD8, CD3, and CD45, and negative for CD4.

The CD8 single positive T cell can be produced by culturing a CD4/CD8 double positive T cell in a culture fluid supplemented with an adrenal cortex hormone agent. The adrenal cortex hormone agent is preferably glucocorticoid or a derivative thereof, such as cortisone acetate, hydrocortisone, fludrocortisone acetate, prednisolone, triamcinolone, methylprednisolone, dexamethasone, betamethasone, and beclometasone dipropionate. As the adrenal cortex hormone agent, dexamethasone is suitably used. The concentration thereof in the culture fluid is, for example, 1 to 100 nM.

A culture fluid used for producing the CD8 single positive T cell is not especially limited, and can be prepared by using a culture fluid used in culture of animal cells as a basal culture fluid, and adding an adrenal cortex hormone agent thereto. Examples of the basal culture fluid include IMDM culture fluid, Medium199 culture fluid, EMEM culture fluid, αMEM culture fluid, DMEM culture fluid, Ham's F12 culture fluid, RPMI1640 culture fluid, Fischer's Neurobasal Medium culture fluid (Life Technologies Corporation), and mixtures of these culture fluids. A serum may be added to the culture fluid, or the culture fluid may be serum-free. The basal culture fluid may contain, if necessary, one or more substances selected from, for example, albumin, insulin, transferrin, selenium, fatty acid, trace elements, 2-mercaptoethanol, thiolglycerol, monothiolglycerol, a lipid, an amino acid, L-glutamine, a non-essential amino acid, a vitamin, a growth factor, a low molecular compound, an antibiotics, an antioxidant, pyruvic acid, a buffer, an inorganic salt, and a cytokine.

The culture fluid used for producing the CD8 single positive T cell may further contain an anti-CD3 antibody, a vitamin C, or a cytokine. Examples of the cytokine include IL-2, IL-7, IL-15, and IL-21. The anti-CD3 antibody is not especially limited as long as it is an antibody specifically recognizing CD3, and an example thereof includes an antibody produced from an OKT3 clone. The concentration of the anti-CD3 antibody in the culture fluid is, for example, 10 to 1000 ng/ml.

In one embodiment of the present invention, an iPS cell clone having a cell surface molecule reactive to a tumor-related antigen introduced thereinto is differentiated into a mature NK cell through a hematopoietic stem cell and an immature NK cell. Maturity of a human NK cell may be determined by using, as a differentiation marker, a cell surface antigen such as CD7, CD16, CD56, or NKG2A. The immature NK cell is a cell at any of all differentiation stages from a hematopoietic stem cell to a mature NK cell, and encompasses, for example, a precursor NK cell and an immature NK cell.

The mature NK cell can be produced from an embryoid body (EB) generated from an iPS cell clone. For producing an embryoid body, an iPS cell clone having been cultured on a dish may be dissociated and transferred to a low-adhesion dish to be cultured using a culture fluid containing a ROCK inhibitor. An example of the culture fluid may include StemFit (R) AK03N (Ajinomoto Co., Inc.) capable of greatly growing an iPS cell kept in an undifferentiated state. An example of the ROCK inhibitor include, but is not limited to, Y-27632. Y-27632 may be added to the culture fluid in a concentration of, for example, 1 to 100 µM. Examples of a method for dissociating an iPS cell clone include a method of physical isolation with a cell scraper or the like, and a method using a dissociation solution having protease activity such as trypsin (for example, TrypLE (R) select, Thermo Fisher Scientific).

The thus obtained embryoid body may be cultured in EB medium containing a cytokine such as BMP4, bFGF, or VEGF. As for the concentrations of these cytokines in the EB medium, for example, that of BMP4 is 1 to 100 ng/mL, that of bFGF is 1 to 100 ng/mL, and that of VEGF is 1 to 100 ng/mL. As the EB medium, for example, StemPro (R)-34 (Thermo Fisher Scientific) containing 2 mL L-glutamine, 400 µM monothioglycerol, 50 µg/mL ascorbic acid-2-phosphate, insulin, transferrin, and selenium may be used.

The number of days for culture is not especially limited as long as a desired cell state is obtained, but from 2 to 6 days after starting the culture, the embryoid body may be cultured in EB medium supplemented with a hematopoietic cytokine cocktail containing, for example, SCF (10 to 100 ng/mL), FLT3L (1 to 100 ng/mL), IL-3 (1 to 100 ng/mL), and TPO (1 to 100 ng/mL). From 10 to 18 days after starting the culture, a differentiated cell may be transferred to a dish coated with FcDLL4 and cultured in EB medium supplemented with a T cell cytokine cocktail containing, for example, FLT3L (1 to 100 ng/mL) and IL-7 (1 to 100 ng/mL). On day 21 to 36 after starting the culture, the hematopoietic cell is differentiated into a CD7/CD45 double positive lymphocyte progenitor cell. When the lymphocyte progenitor cell is co-cultured by using, as a feeder cell, allogeneic PBMC (peripheral blood mononuclear cell) having been irradiated with radiation to be expansion-cultured with PHA (phytohemagglutinin), and thus, an NK cell that expresses a cell surface molecule reactive to a tumor-related antigen can be obtained. The thus produced mature NK cell is negative for CD3, CD4, CD5, CD8α, and CD8β, and positive for CD56, CD159a, CD161, CD226, CD314, CD336, and CD337.

### [Introduction of Cytokine Gene into Immune Cell Expressing Cell Surface Molecule Reactive to Tumor-related Antigen]

In the present invention, to the hematopoietic stem cell expressing a cell surface molecule reactive to a tumor-related antigen, the immature immune cell, or the mature immune cell obtained as described above, nucleic acids encoding cytokines are introduced from outside the cell. The cytokines are IL-15, IL-12, IL-18 and IL-21, and the nucleic acids encoding these are preferably human-derived nucleic acids, and may be naturally-derived nucleic acids, or artificially synthesized nucleic acids. Sequence information of these nucleic acids is available from known literature and databases such as NCBI (http://www.ncbi.nlm.nih.gov/guide/) or the like.

In the present invention, a cytokine gene to be introduced into the hematopoietic stem cell, the immature immune cell, or the mature immune cell is a nucleic acid encoding IL-15, and in one embodiment, is nucleic acids encoding IL-15 and IL-12, nucleic acids encoding IL-15 and IL-18, or nucleic acids encoding IL-15 and IL-21. cDNAs encoding these cytokines may be prepared to be incorporated into expression vectors.

As the expression vector, a viral vector and a non-viral vector can be used. Examples of the viral vector include a retrovirus, a lentivirus, an adenovirus, an adeno-associated virus, a herpes virus, and a Sendai virus, as well as an animal cell expression plasmid. A retrovirus or a lentivirus may be suitably used. In performing infection with a retrovirus or a lentivirus, a spin infection method or the like may be employed. An example of the non-viral vector may include a piggyBac (R) vector that is a transposon vector. Examples a gene transfer method for a non-viral vector include a lipofection method, a liposome method, a calcium phosphate coprecipitation method, a DEAE dextran method, a microinjection method, and an electroporation method.

The gene transfer of the cytokine gene may be performed in an arbitrary region of a group of gene loci specifically expressed in the hematopoietic stem cell, the immature immune cell, or the mature immune cell, or may be performed on a safe harbor gene locus for preventing modification or disruption of existing genes by the introduced gene. An example of the safe harbor includes AAVS1 (Adeno-associated virus integration site 1) region in the human genome. Examples of a method for introducing a gene targeting the safe harbor include CRISPR/Cas9 method and TALEN method.

When two cytokine genes are to be introduced, the two cytokine genes may be incorporated into one expression vector, or the two cytokine genes may be incorporated respectively into different expression vectors. In other words, two nucleic acids encoding two cytokine genes may be transcribed with one promoter by using IRES (internal ribosome entry site) or 2A self-cleaving peptide, or may be transcribed respectively with different promoters. When two cytokine genes are incorporated into one expression vector, namely, when nucleic acids encoding IL-15 and IL-12, nucleic acids encoding IL-15 and IL-18, or nucleic acids encoding IL-15 and IL-21 are to be incorporated, one nucleic acid may be disposed either upstream or downstream of the other nucleic acid.

### [Pharmaceutical Containing Cell of Present Invention]

A pharmaceutical containing the cell of the present invention can be used as an agent for preventing or treating cancer in a mammal. The pharmaceutical of the present invention may be produced by a method usually employed in the art of formulation technology. The pharmaceutical of the present invention may contain a pharmaceutically acceptable additive. Examples of the additive include a cell culture fluid, saline, and an appropriate buffer (such as phosphate buffer).

The pharmaceutical of the present invention may be produced by suspending the cell of the present invention in saline, an appropriate buffer (such as phosphate buffer) or the like. For exhibiting a desired therapeutic effect, a dosage may contain, for example, 1 × 10⁷ or more, 1 × 10⁸ or more, or 1 × 10⁹ or more cells. The content of the cell may be adjusted in consideration of the sex, the age, the weight, and the condition of the affected part of a subject of the administration, the condition of cells, and the like. The pharmaceutical of the present invention may contain, in addition to the cell of the present invention, dimethyl sulfoxide (DMSO), serum albumin, and the like for purpose of protecting the cell. Besides, the pharmaceutical may contain an antibiotics and the like for preventing bacterial contamination. In addition, the pharmaceutical may contain a vitamin, a cytokine, and the like for activating the cell and enhancing the differentiation. The pharmaceutical of the present invention may further contain another pharmaceutically acceptable component (such as a carrier, an excipient, a disintegrating agent, a buffer, an emulsifier, a suspending agent, a soothing agent, a stabilizer, a preservative, an antiseptic, or saline).

The pharmaceutical containing the cell of the present invention can be cryopreserved. In cryopreservation, the preservation temperature is not especially limited as long as it is suitable for preserving cells. The temperature is, for example, -20°C, -80°C, or -120 to -196°C, and is preferably -150°C or lower. In the cryopreservation, the cell may be stored in an appropriate container such as a cryopreservation vial, or a cryopreservation bag.

The pharmaceutical of the present invention is used for preventing or treating cancer. Examples of the cancer include, but are not limited to, ovarian cancer, hepatoblastoma, hepatocellular carcinoma, stomach cancer, esophageal cancer, pancreatic cancer, renal cell cancer, breast cancer, malignant melanoma, non-small cell lung cancer, cervical cancer, glioblastoma, prostate cancer, neuroblastoma, chronic lymphocytic leukemia, papillary thyroid cancer, colorectal cancer, head and neck cancer, brain tumor, multiple myeloma, and B-cell non-Hodgkin's lymphoma.

The cell of the present invention can kill a cell expressing a tumor-related antigen, and hence can be used as a cytotoxic agent for a cell expressing a tumor-related antigen. The cytotoxic agent can be produced and used in the same manner as the pharmaceutical.

Now, the present invention will be described by way of examples, and it is noted that the present invention is not limited to the following examples.

### [Statistical Analysis]

A statistically significant difference described herein was analyzed with GraphPad Prism 8 software by one-way analysis of variance (one-way ANOVA) employing Tukey's multiple comparison test (*P<0.05; **P<0.01; ***P<0.005; ****P<0.001).

### Example 1

### [Production of iPS-T cell having CAR Reactive to GPC3 introduced thereinto]

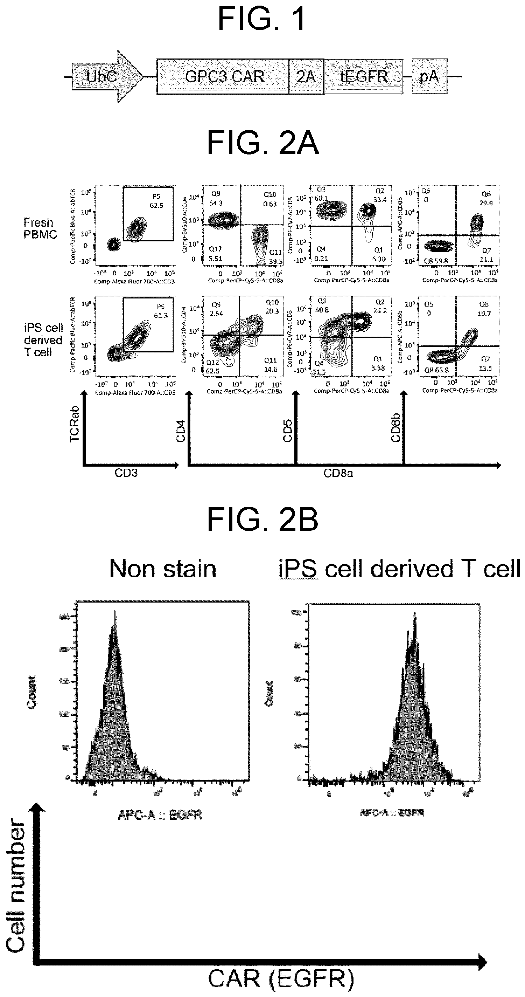

A lentivirus expression vector illustrated in Figure 1 was used to introduce, into an iPS cell, a CAR reactive to GPC3 (Glypican-3) that is a tumor-related antigen (GPC3-CAR). A clone cell was obtained from an iPS cell in which the gene transfer had been confirmed, and was differentiated into a T cell. Expression of TCR α chain and β chain, CD3, CD4, CD5, and CD8 α chains and β chains that are surface markers of the differentiated cell (GPC3-reactive iPS-CAR-T cell) was analyzed by flow cytometry. As a result, it was confirmed that the GPC3-reactive iPS-CAR-T cell was a CD8 α chain/β chain double positive cell (Figure 2A). Besides, as a result of flow cytometry of the CAR, a mature GPC3-reactive iPS-CAR-T cell expressed EGFR gene linked to a T2A sequence, and hence, was found to have expressed the CAR (Figure 2B).

### Example 2

### [Introduction of Cytokine Gene into GPC3-reactive iPS-CAR-T Cell]

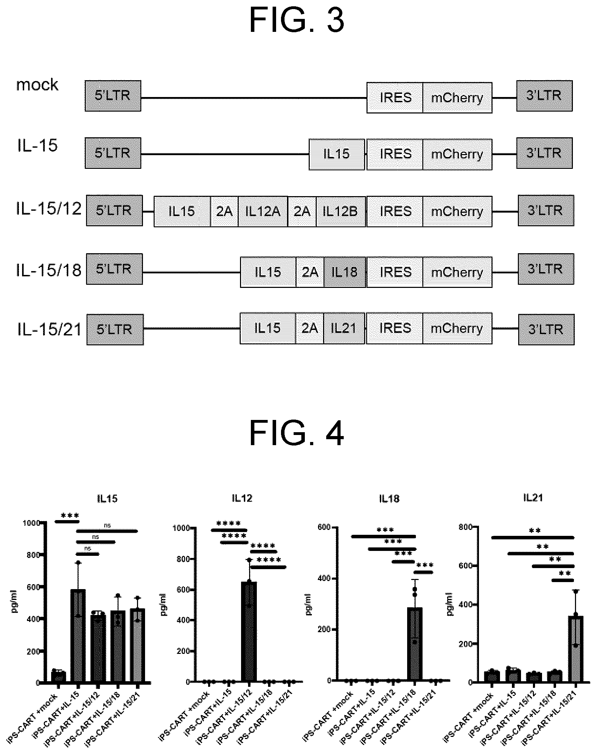

A retrovirus expression vector illustrated in Figure 3 was used to introduce cytokine (IL-15, IL-12, IL-18, and IL-21) genes into the CD8 α chain/β chain double positive GPC3-reactive iPS-CAR-T cell produced in Example 1. The expression of fluorescent protein mCherry, which had been incorporated into the expression vector through a bond to an IRES sequence, was evaluated by flow cytometry, and as a result, the mCherry expression was found at approximately the same level in the cells having any of the vector constructs introduced thereinto.

The GPC3-reactive iPS-CAR-T cells incorporating the cytokine genes were cultured for 48 hours, the production of the cytokines in the thus obtained culture supernatants was measured by the ELISA method, and the results are illustrated in Figure 4. Terms in the drawing are used in the following meanings: iPS-CART + mock means a mock cell; iPS-CART + IL-15 means a cell having IL-15 gene introduced thereinto; iPS-CART + IL-15/12 means a cell having IL-15 and IL-12 gene introduced thereinto ; iPS-CART + IL-15/18 means a cell having IL-15 and IL-18 gene introduced thereinto; and iPS-CART + IL-15/21 means a cell having IL-15 and IL-21 gene introduced thereinto. The test was performed on independent three samples, and the results are shown as mean ± standard error of the cytokine concentration in the cell culture fluid.

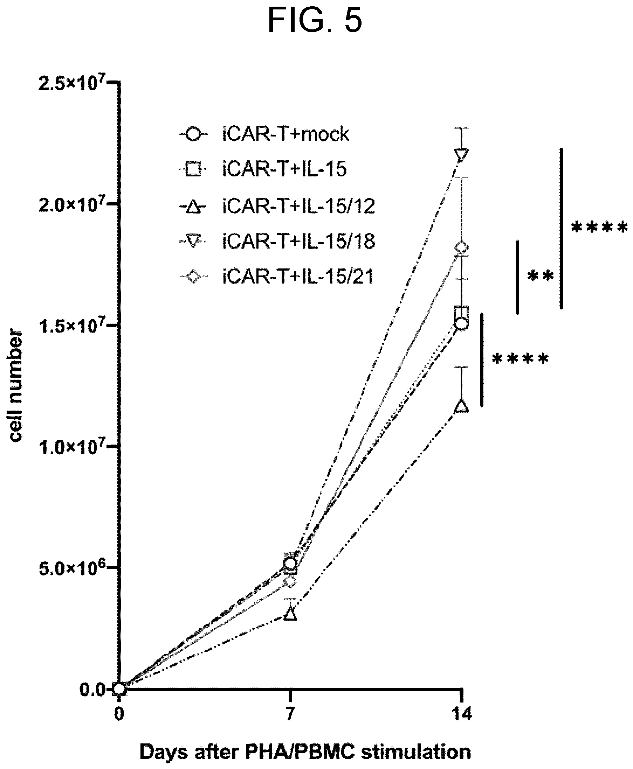

The above GPC3-reactive iPS-CAR-T cells having the cytokine genes introduced thereinto showed significant increase in the cytokine production corresponding to the cytokine genes introduced. Next, the above GPC3-reactive iPS-CAR-T cells having the cytokine genes introduced thereinto were stimulated with PHA (phytohemagglutinin) and PBMC (peripheral blood mononuclear cell) in the absence of CAR specific stimulation to examine the relationship between the cytokine produced by the cell and the cell proliferative capacity. The results are illustrated in Figure 5. The meanings of the terms in the drawing are as described above and are the same as in Figure 4. The test was performed on independent three samples, and the results are shown as mean ± standard error of the cell number. As a result, the cell proliferative capacity was significantly increased in the cell expressing IL-15 and IL-18, and the cell expressing IL-15 and IL-21. On the other hand, the cell proliferative capacity was significantly reduced in the cell expressing IL-15 and IL-12. The cell proliferative capacity was hardly affected in the cell expressing only IL-15. These results revealed that the cell proliferative capacity is affected when a cytokine gene is introduced into a GPC3-reactive iPS-CAR-T cell to express the cytokine.

### Example 3

### [Cytotoxic Activity of Cytokine-Expressing GPC3-reactive iPS-CAR-T Cell]

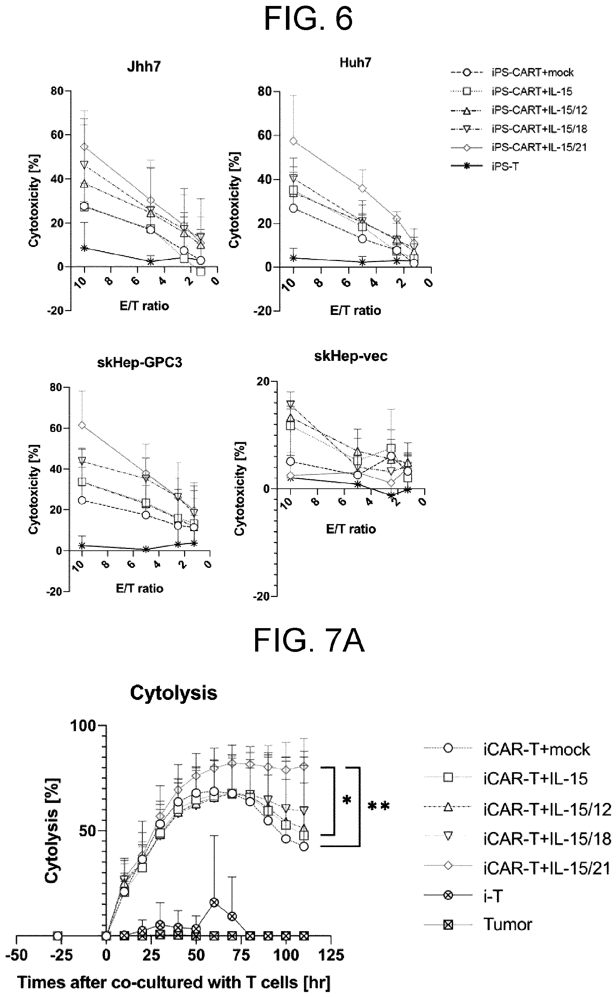

Cytotoxic activity was examined by non-radioactive cytotoxicity assay by using the cytokine-expressing GPC3-reactive iPS-CAR-T cell produced in Example 2 as an effector cell (E), using, as target cells (T), JHH-7 and HuH-7, that is, GPC3 positive human liver cancer cell lines, and human liver cancer-derived cell line SK-HEP-1 having GPC3 gene introduced thereinto (skHep-GPC3), and using SK-HEP-1 not having GPC3 introduced thereinto (skHep-vec) as a control. The results are illustrated in Figure 6. Terms in the drawing are used in the following meanings: iPS-CART + mock (mock cell), iPS-CART + IL-15 (cell having IL-15 gene introduced thereinto), iPS-CART + IL-15/12 (cell having IL-15 and IL-12 genes introduced thereinto), iPS-CART + IL-15/18 (cell having IL-15 and IL-18 genes introduced thereinto), iPS-CART + IL-15/21 (cell having IL-15 and IL-21 genes introduced thereinto), and control iPS-T cell (iPS-T). The test was performed on independent four samples, and the results are shown as mean ± standard error.

The IL-15/21-expressing GPC3-reactive iPS-CAR-T cell (iPS-CART + IL-15/21) exhibited cytotoxic activity against all the target cells used (JHH-7, HuH-7, and skHep-GPC3), and the effect was obviously increased as compared with that of the mock cell not having the cytokine gene introduced thereinto (Figure 6). Besides, iPS-CART + IL-15/21 exhibited the strongest cytotoxic activity among the examined effector cells. The above cells expressing IL-15 and IL-18, or IL-15 and IL-12 were found to have stronger cytotoxic activity than the mock cell. The above cell expressing IL-15 exhibited cytotoxic activity against HuH-7 and skHep-GPC3. Non-specific cytotoxic activity that could be caused when a cytokine was expressed was not also observed. It was found, based on these results, that the GPC3-reactive iPS-CAR-T cell retains specific cytotoxic activity against GPC3 even when a cytokine is expressed. The cytotoxic activity of the above cytokine-expressing GPC3-reactive iPS-CAR-T cell against skHep-vec not expressing GPC3 was not observed.

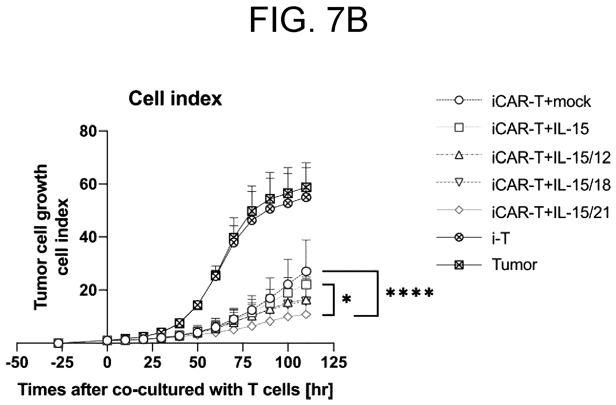

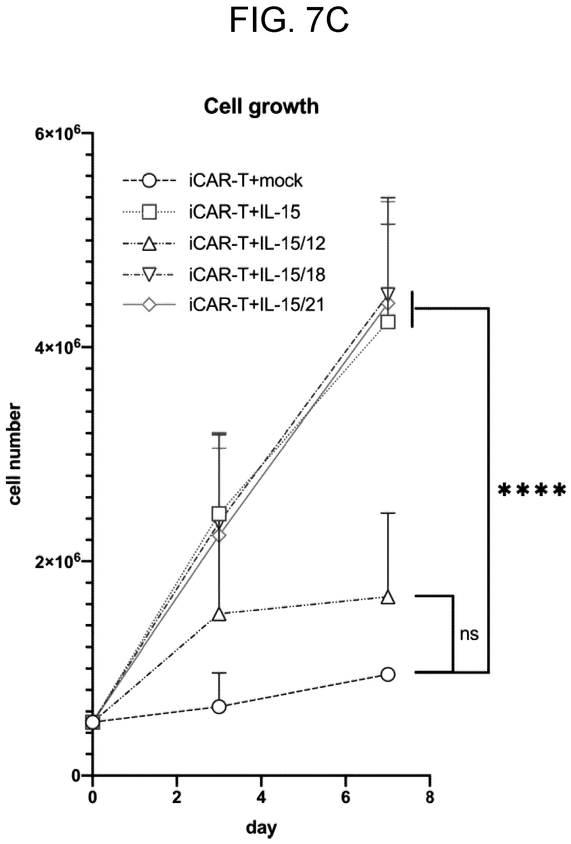

The cytokine-expressing GPC3-reactive iPS-CAR-T cell and the skHep-GPC3 cell were co-cultured, and results of cytotoxicity against the skHep-GPC3 cell measured over time are illustrated in Figure 7A, results of the proliferation of the skHep-GPC3 cell measured over time are illustrated in Figure 7B, and results of proliferation of the cytokine-expressing GPC3-reactive iPS-CAR-T cell measured over time are illustrated in Figure 7C. In all the measurements, a real-time cell analyzer xCELLigence (Agilent) was used. In Figures 7A and 7B, "Tumor" indicates the results in the absence of the iPS-CAR-T cell, and "iPS-T" indicates a control iPS-T cell having no CAR molecule. In each of Figures 7A, 7B, and 7C, the test was performed on independent three samples, and the results are shown as mean ± standard error.

The cytotoxic activity over time against the skHep-GPC3 cell was not reduced in the IL-15- and IL-21-expressing GPC3-reactive iPS-CAR-T cell for 100 hours or more, and was significantly strongly retained as compared with that of the mock cell (Figure 7A). Correspondingly, the IL-15- and IL-21-expressing GPC3-reactive iPS-CAR-T cell retained the effect of inhibiting the proliferation of the skHep-GPC3 cell for 100 hours or more, and inhibited the proliferation significantly strongly as compared with the mock cell (Figure 7B). This effect was found also in the GPC3-reactive iPS-CAR-T cells expressing IL-15 and IL-18, IL-15 and IL-12, or only IL-15, but was weak in these. It was also revealed that the cytotoxic activity disappeared in about 70 hours after the co-culture.

In the co-culture with the skHep-GPC3 cell, the GPC3-reactive iPS-CAR-T cells expressing IL-15, IL-15 and IL-18, or IL-15 and IL-21 were increased in the cell number time-dependently up to day 7 of the co-culture, but the IL-15- and IL-12-expressing GPC3-reactive iPS-CAR-T cell was not continuously increased in the cell number. Accordingly, it is suggested that the cytotoxic activity and the cell proliferation inhibitory effect of the cytokine-expressing GPC3-reactive iPS-CAR-T cell do not simply depend on the cell number.

### Example 4

### [Anti-apoptotic Activity of Cytokine-expressing GPC3-reactive iPS-CAR-T Cell]

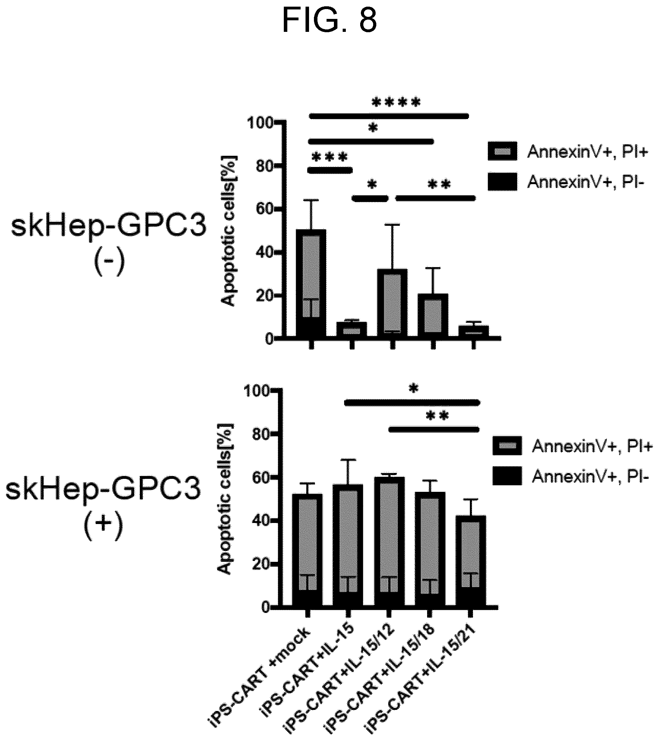

The cytokine-expressing GPC3-reactive iPS-CAR-T cells produced in Example 2 were cultured in the presence or absence of human liver cancer-derived cell line SK-HEP-1 having the GPC3 gene introduced thereinto (skHep-GPC3), and stained with Annexin V and PI (Propidium Iodide) for analysis by flow cytometry, and thus apoptosis cells were detected. The results are illustrated in Figure 8. The test was performed on independent three samples, and the results are shown as mean ± standard error.

In the flow cytometry analysis, late apoptotic cells can be stained with both Annexin V and PI. It was revealed that the number of the apoptotic cells in the GPC3-reactive iPS-CAR-T cells expressing IL-15, IL-15 and IL-18, or IL-15 and IL-21 was significantly smaller than that of the mock cell in the absence of skHep-GPC3 cell, that is, an antigen cell (upper graph of Figure 8). It was revealed that the number of late apoptotic cells in the IL-15- and IL-21-expressing GPC3-reactive iPS-CAR-T cells was significantly smaller than that of the GPC3-reactive iPS-CAR-T cells expressing IL-15, and IL-15 and IL-21 in the presence of the sk-Hep-GPC cells (lower graph of Figure 8). These results suggest that particularly the IL-15- and IL-21-expressing cell among the cytokine-expressing GPC3-reactive iPS-CAR-T cells has strong anti-apoptotic activity within a tumor tissue, and retains the antitumor effect.

### Example 5

### [In Vitro Migration Ability of Cytokine-expressing GPC3-reactive iPS-CAR-T Cell]

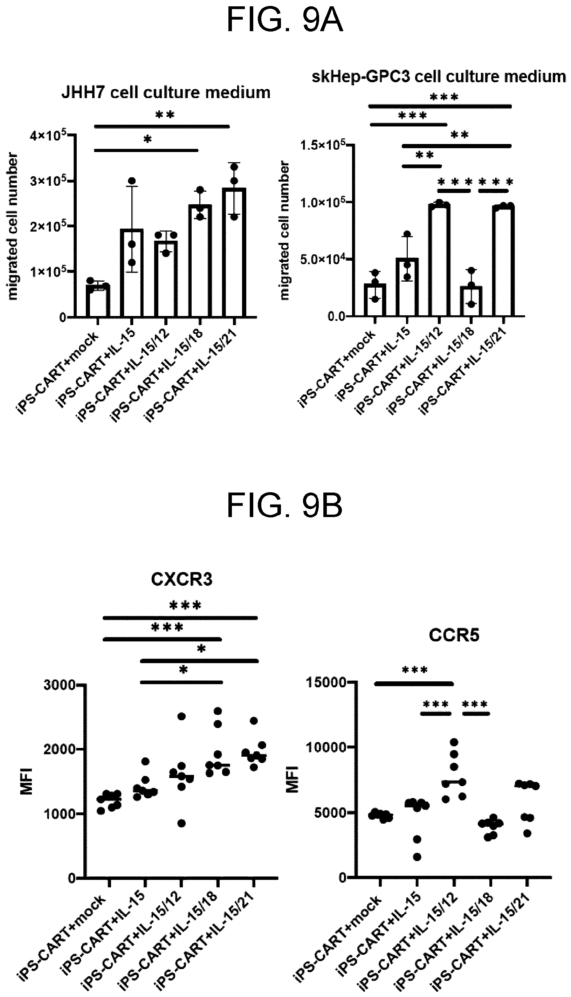

The migration ability of the cytokine-expressing GPC3-reactive iPS-CAR-T cells produced in Example 2 was evaluated by Transwell (R) assay using culture supernatants obtained by culturing JHH-7 cell and skHep-GPC3 cell for 2 days. The assay was performed as follows: The culture supernatant was added to a plate well of Transwell, a plate insert (polycarbonate membrane having a pore size of 5 µm) to which the iPS-CAR-T cell had been added was inserted into the well for culturing the cell for 2 hours, and the migrated cell number was measured. The results are illustrated in Figure 9A. The test was performed on independent three samples, and the results are shown as mean ± standard error. When the culture supernatant of JHH-7 cell was used, the migration ability of the cytokine-expressing GPC3-reactive iPS-CAR-T cells was enhanced, and in particular, the migration ability of the iPS-CAR-T cells expressing IL-15 and IL-21, or IL-15 and IL-18 was significantly enhanced as compared with that of the mock cell. On the other hand, when the culture supernatant of skHep-GPC3 cell was used, the migration ability of the iPS-CAR-T cells expressing IL-15 and IL-12, or IL-15 and IL-21 was significantly enhanced as compared with that of the mock cell.

CCR5 and CXCR3 play a significant role in cell migration, and are representative chemokine receptors in migration particularly of initial CD8 positive T cell to solid tumors. Therefore, in the cytokine-expressing GPC3-reactive iPS-CAR-T cells, the expression of CXCR3 and CCR5 was analyzed by flow cytometry. The results are illustrated in Figure 9B. The test was performed on independent seven samples, and the results are shown as mean ± standard error. As for the chemokine receptor expression, it was revealed, based on the analysis results, that the expression of CCR5 was significantly increased in the IL-15- and IL-12-expressing iPS-CAR-T cell as compared with that in the mock cell, and that of CXCR3 was significantly increased in the iPS-CAR-T cells expressing IL-15 and IL-18, or IL-15 and IL-21 compared with that in the mock cell.

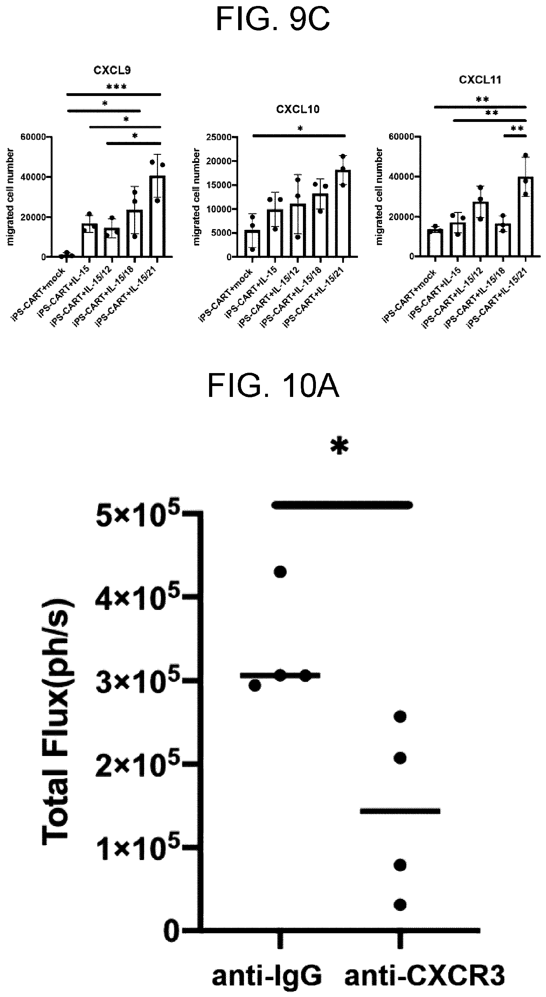

As described above, the expression of CXCR3 was increased in the iPS-CAR-T cells expressing IL-15 and IL-18, or IL-15 and IL-21, and therefore, migration ability to CXCL9, CXCL10, and CXCL11, which are ligands of CXCR3, and are increased in secretion in various tumors, was examined. The migration ability was evaluated by Transwell (R) assay as described above. The results are illustrated in Figure 9C. The test was performed on independent three samples, and the results are shown as mean ± standard error. As a result, it was revealed that the migration ability of the IL-15- and IL-21-expressing GPC3-reactive iPS-CAR-T cell to CXCL9, CXCL10, and CXCL11 was significantly enhanced as compared with that of the mock cell and the IL-15-expressing GPC3-reactive iPS-CAR-T cell.

### Example 6

### [In Vivo Migration Ability of IL-15- and IL-21-Expressing GPC3-reactive iPS-CAR-T Cell]

The results of Example 5 suggested that the IL-15 and IL-21 (IL-15/21) expressing GPC3-reactive iPS-CAR-T cell migrates to solid tumor. Therefore, a tumor-bearing mouse was used to examine a migration property to solid tumor of the IL-15/21-expressing GPC3-reactive iPS-CAR-T cell produced in Example 2.

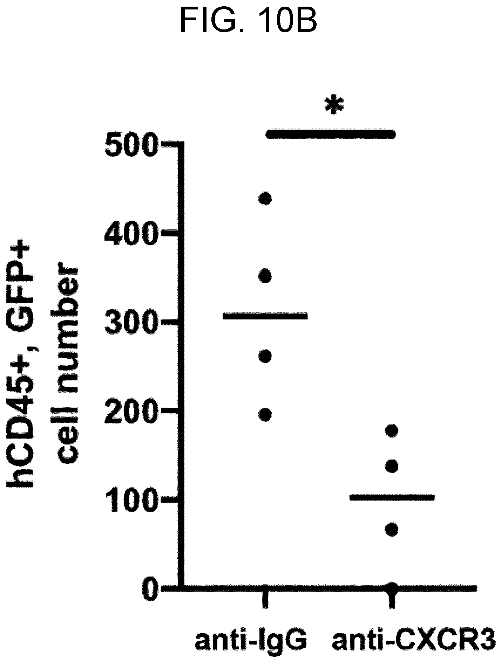

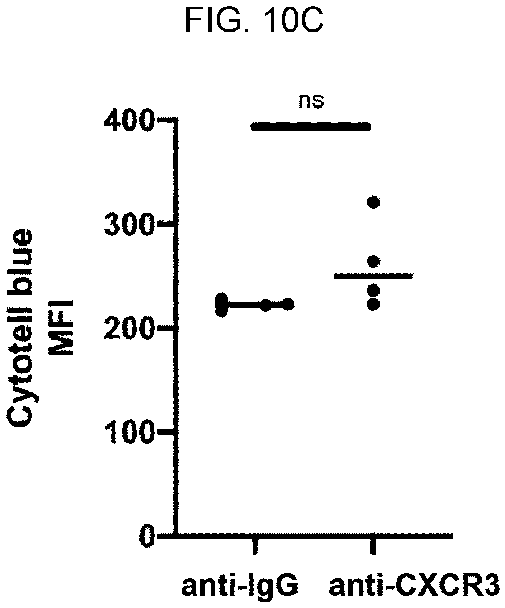

NSG (NOD-SCID IL2Rγc^{null}) mice were purchased from OrientalBio Co., Ltd. On day 0, human liver cancer cell line JHH-7 (0.5 × 10⁶ cells) was subcutaneously injected into a left dorsal part of each NSG mouse. On day 14, GPC3-reactive iPS-CAR-T cells having the IL-15/21 gene introduced thereinto (1 × 10⁷ cells), which had been treated with an anti-human CXCR3 antibody, or an anti-human IgG₁ antibody as a control, were intravenously administered. On day 16, the iPS-CAR-T cells were observed by *in vivo* imaging using luciferase luminescence. As a result, when the iPS-CAR-T cell having been treated with the anti-human CXCR3 antibody was administered, luciferase luminescence around the mouse tumor was significantly reduced as compared with a case where the cell having been treated with the anti-human IgG₁ antibody was administered (Figure 10A). Therefore, the mouse was dissected, and the number of the iPS-CAR-T cells having infiltrated into the mouse tumor was analyzed by measuring human CD45 positive and GFP positive cells. As a result, it was confirmed that the number of cells infiltrating into the tumor is significantly reduced by the treatment with the anti-human CXCR3 antibody (Figure 10B). It was confirmed, by labeling the iPS-CAR-T cells with a cell proliferation monitoring reagent, CytoTell Blue (R) (AAT Bioquest, Inc.) before the administration to the mouse, that the reduction in the number of cells infiltrating into the tumor by the treatment with the anti-human CXCR3 antibody was not caused due to deterioration of cell proliferative capacity. Specifically, there was not a significant difference in mean fluorescence intensity of CytoTell Blue for detecting cell division between the group treated with the anti-human CXCR3 antibody and the group treated with the anti-human IgG₁ antibody (Figure 10C).

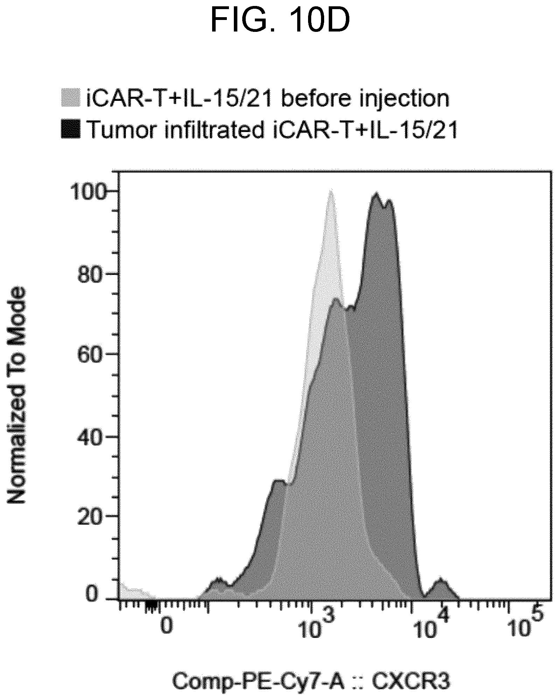

The CXCR3 expression of the GPC3-reactive iPS-CAR-T cell having the IL-15/21 gene introduced thereinto, infiltrated into the mouse tumor was analyzed by flow cytometry, and the result was compared with that obtained before the administration to the mouse (Figure 10D). As a result, it was revealed that the expression of CXCR3 was enhanced in the iPS-CAR-T cells having infiltrated into the tumor. It is understood from these results that the IL-15/21-expressing GPC3-reactive iPS-CAR-T cell migrates into the mouse tumor CXCR3-dependently. It is suggested that the intravenously administered IL-15/21-expressing GPC3-reactive iPS-CAR-T cell migrates to solid tumor, accumulates in the tissue of the tumor, and then exhibits the antitumor effect.

### Example 7

[Analysis of Molecular Mechanism of CXCR3 Expression Enhancement in IL-15- and IL-21-Expressing GPC3-reactive iPS-CAR-T Cell]

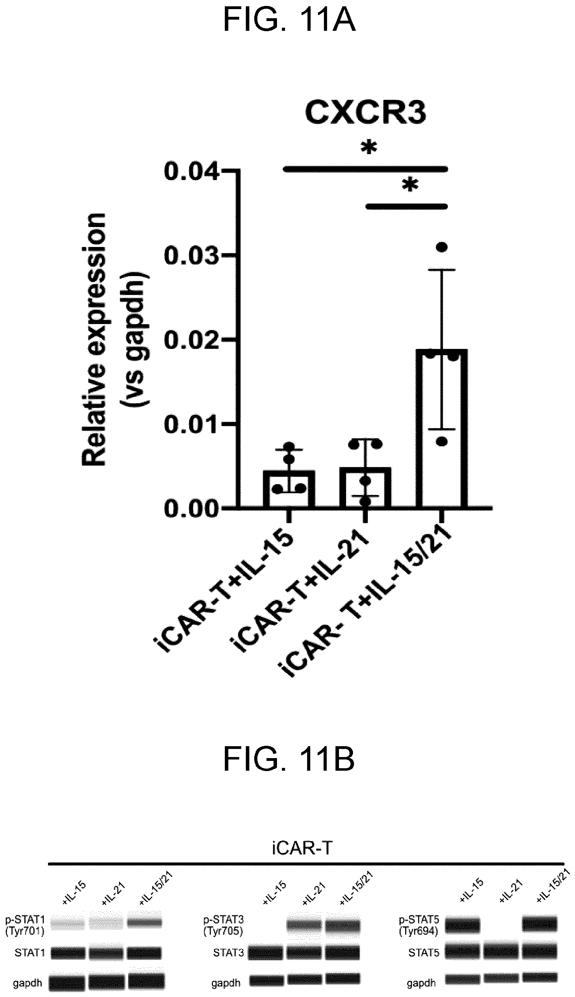

The molecular mechanism that the CXCR3 expression in the IL-15 and IL-21 (IL-15/21) expressing GPC3-reactive iPS-CAR-T cell controls the migration was analyzed. First, the CXCR3 expression level in the GPC3-reactive iPS-CAR-T cell expressing IL-15, IL-21, or IL-15/21 produced in Example 2 was evaluated by quantitative PCR as a relative expression level to GAPDH (glyceraldehyde-3-phosphate dehydrogenase). As a result, it was found that the CXCR3 expression was significantly enhanced in the IL-15/21-expressing cell as compared with that in the IL-15- or IL-21-expressing cell (Figure 11A). In the drawing, the IL-15-expressing iPS-CAR-T cell is indicated as "iCAR-T + IL-15", the IL-21-expressing iPS-CAR-T cell is indicated as "iCAR-T + IL-21", and the IL-15/21-expressing iPS-CAR-T cell is indicated as "iCAR-T + IL-15/21".

It has been reported that IL-15 and IL-21 phosphorylate a tyrosine residue of transcription factors STAT1, STAT3, and STATS to activate the transcription (O'Shea J. J. et al., Annu Rev Med. 2015; 66: 311-328). Therefore, the expression of phosphorylated STAT1, STAT3, and STATS in the iCAR-T + IL-15, the iCAR-T + IL-21, and the iCAR-T + IL-15/21 was analyzed by Western blotting. For the detection, the following antibodies were used: GAPDH Rabbit mAb (Clone: 14C10), Phospho-Stat1 (Tyr701) Rabbit mAb (Clone: 58D6), Phospho-Stat3 (Tyr705) Rabbit mAb (Clone: D3A7), Phospho-Stat5 (Tyr694) Rabbit mAb (Clone: C11C5), Stat1 Rabbit mAb (Clone: D1K9Y), Stat3 Rabbit mAb (Clone: D3Z2G), Stat5 Rabbit mAb (Clone: D2O6Y). These antibodies were purchased from Cell Signaling TECHNOLOGY. The relative expression levels of STAT1, STAT3, and STATS to GAPDH were evaluated by image analysis using Compass software.

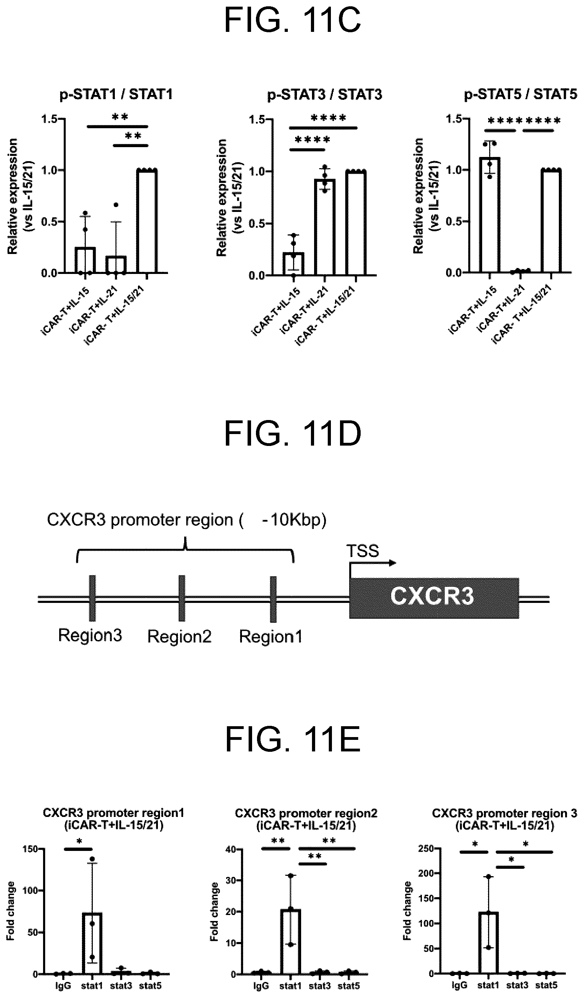

The results of the analysis by Wester blotting are illustrated in Figures 11B and 11C. As a result of image analysis of stained images in Figure 11B, it was revealed that the phosphorylated STAT1, STAT3, and STATS were significantly increased in the iCAR-T + IL-15/21 as compared with those in the other cells (Figure 11C). In particular, the expression of the phosphorylated STAT1 in the iCAR-T + IL-15/21 was significantly increased as compared with those in both the iCAR-T + IL-15 and the iCAR-T + IL-21.

In order to verify direct involvement of STAT1, STAT3, and STATS in the transcription of CXCR3, chromatin immunoprecipitation (ChIP)-qPCR was performed on the promoter region of CXCR3 by using an anti-STAT1 antibody, an anti-STAT3 antibody, and an anti-STAT5 antibody. An anti-rabbit IgG antibody (#2729, Cell Signaling TECHNOLOGY, Inc.) was used as a control antibody. Figure 11D illustrates three regions to be respectively amplified by three different primer pairs used in the PCR in the promoter region of CXCR3. As a result of the ChIP-qPCR, it was revealed that STAT1 was significantly bound to the CXCR3 promoter region (Figure 11E).

### Example 8

### [In Vivo Kinetics of GPC3-reactive iPS-CAR-T Cell Having Cytokine Gene Introduced thereinto in Mice Transplanted with Human Liver Cancer-derived Cell Line SK-HEP-1]

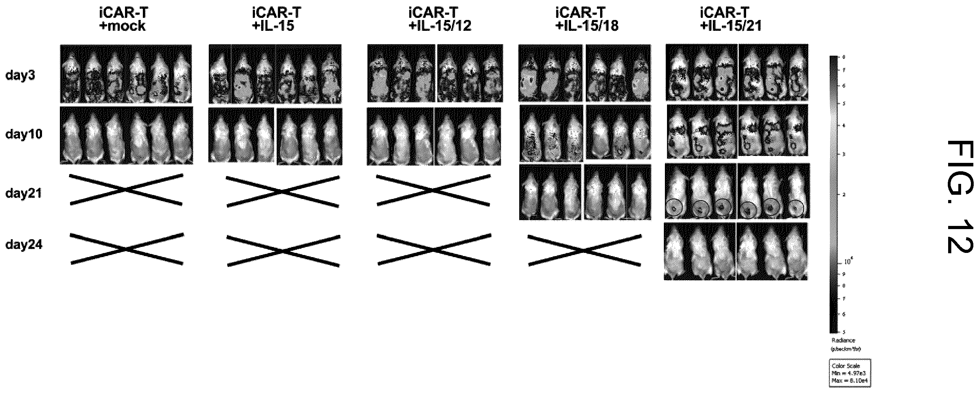

Human liver cancer-derived cell line SK-HEP-1 having the GPC3 gene introduced thereinto (2.5 × 10⁶ cells) was subcutaneously injected into a left dorsal part of each NSG mouse. After confirming a tumor nodule in the mouse, luciferase-expressing GPC3-reactive iPS-CAR-T cells having the cytokine gene introduced thereinto (5 × 10⁶ cells, see Example 2) were intravenously administered. Thereafter, the luciferase luminescence was observed over time by *in vivo* imaging. The results are illustrated in Figure 12. On day 14 after the administration of the iPS-CAR-T cells having the cytokine gene introduced thereinto, the luciferase luminescence was not observed around the mouse tumor in the groups administered with the mock cell, and the iPS-CAR-T cells expressing IL-15, or IL-15 and IL-12 (IL-15/12). On the other hand, it was confirmed that the luciferase luminescence was retained around the tumor when the iPS-CAR-T cells expressing IL-15 and IL-18 (IL-15/18), or IL-15 and IL-21 (IL-15/21) were administered. It was revealed that when the IL-15/21-expressing iPS-CAR-T cell was administered in particular, the luciferase luminescence was retained for the longest period of time.

Next, after administering the GPC3-reactive iPS-CAR-T cells having the cytokine gene introduced thereinto, the insides of the mouse tumors were immunofluorescent stained with an anti-CD3 antibody, an anti-Ki67 antibody, and DAPI. In the mouse tumor administered with the IL-15/21-expressing iPS-CAR-T cell, there were a larger number of CD3 positive cells than in the tumors administered with the iPS-CAR-T cells expressing IL-15, IL-15/12, and IL-15/18. Since a CD3 positive cell is regarded as a marker of an iPS-CAR-T cell, it is revealed that a larger number of iPS-CAR-T cells infiltrate into the tumor by expressing IL-15/21. In other words, it is suggested that the IL-15/21-expressing iPS-CAR-T cell has stronger cytotoxic activity to the tumor than the iPS-CAR-T cells expressing the other cytokines. In the tumor tissue administered with the IL-15/21-expressing iPS-CAR-T cell, a CD3 and Ki67 double positive cell was present. The presence of a CD3 and Ki67 double positive cell suggests that the IL-15/21-expressing iPS-CAR-T cell has cell proliferative capacity.

### Example 9

### [Effects of GPC3-reactive iPS-CAR-T cells Having Cytokine Gene Introduced thereinto on Tumor Volume and Survival Rate of Mice Transplanted with Human Liver Cancer Cell Line JHH-7]

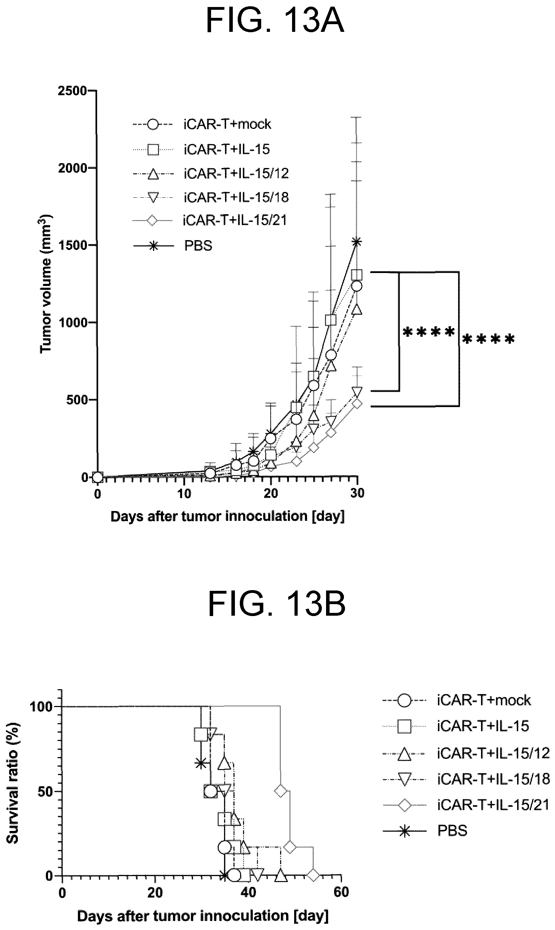

On day 0, human liver cancer cell line JHH-7 (0.5 × 10⁶ cells) was subcutaneously injected into a left dorsal part of each NSG mouse. Next, on days 3 and 10, the GPC3-reactive iPS-CAR-T cells having the cytokine gene introduced thereinto (5 x 10⁶ cells, see Example 2) suspended in PBS (phosphate buffered saline) were intravenously administered. Thereafter, change of the tumor volumes and survival rates of the mice were observed. A tumor volume (V) was calculated based on the length (L, longest dimension) and width (W, shortest dimension) of the tumor in accordance with an expression, V = LW²/2. The results of the tumor volumes are illustrated in Figure 13A. 8 mice under each condition were used, and the result are shown as mean ± standard error. The results of the survival rates are illustrated in Figure 13B.

As for the tumor volume, the iPS-CAR-T cells expressing IL-15 and IL-21, or the IL-15 and IL-18 strongly inhibited the tumor progression (Figure 13A). It was revealed that the IL-15- and IL-21-expressing iPS-CAR-T cell also significantly improves the survival rate, and definite antitumor activity was thus confirmed (Figure 13B).

### Example 10

### [Effects of GPC3-reactive iPS-CAR-T cells Having Cytokine Gene Introduced thereinto on Tumor Volume and Survival Rate of Mice Transplanted with Human Liver Cancer Cell Line SK-HEP-1]

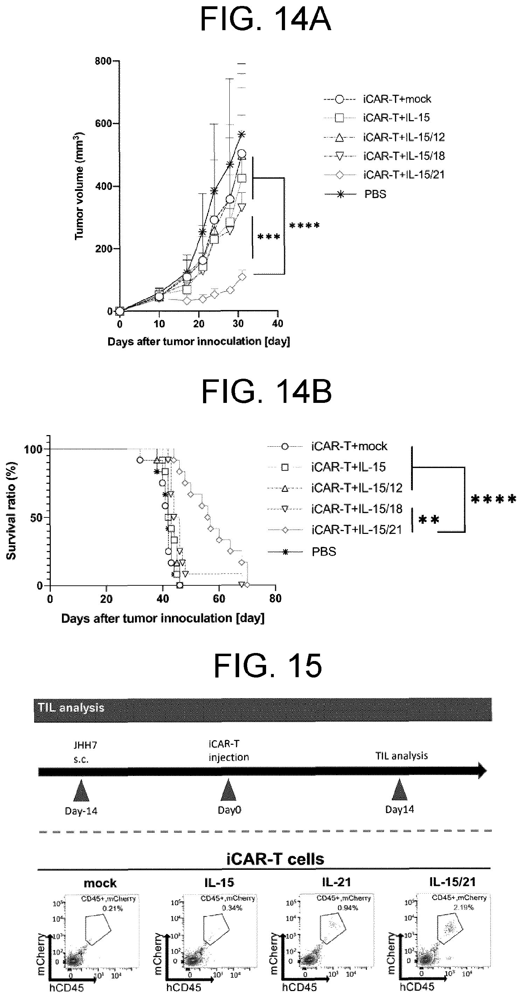

On day 0, human liver cancer-derived cell line SK-HEP-1 having the GPC3 gene introduced thereinto (2.5 × 10⁶ cells) was subcutaneously injected into a left dorsal part of each NSG mouse. Next, on days 10 and 17, the GPC3-reactive iPS-CAR-T cells having the cytokine gene introduced thereinto (5 x 10⁶ cells, see Example 2) suspended in PBS (phosphate buffered saline) were intravenously administered. Thereafter, change of the tumor volumes and survival rates of the mice were observed. The tumor volume was calculated in the same manner as in Example 9. The results of the tumor volume are illustrated in Figure 14A. The result are shown as mean ± standard error ( n = 10). The results of the survival rates are illustrated in Figure 14B ( n = 12).

As for the tumor volume, the iPS-CAR-T cells expressing IL-15 and IL-21, or the IL-15 and IL-18 strongly inhibited the tumor progression (Figure 14A). In particular, the IL-15- and IL-21-expressing iPS-CAR-T cell exhibited extremely strong antitumor effect. Besides, the IL-15- and IL-21-expressing iPS-CAR-T cell was also excellent in improvement of the survival rate, and exhibited excellent prevention or treatment effect on solid tumor (Figure 14B).

### Example 11

### [Comparison of Amounts of GPC3-reactive iPS-CAR-T Cells Having Cytokine Gene Introduced thereinto Present in Tumor]

Each of IL-15, IL-21, or IL-15 and IL-21 (IL-15/21) genes were introduced into and expressed in iPS-CAR-T cells, which were obtained by introducing a glypican 3 (GPC3) specific CAR into an iPS-derived T cell. GPC3 positive human liver cancer cell line JHH-7 was subcutaneously inoculated to immunodeficient mice (NSG mice), and after 14 days, each of the above iCAR-T cells having the cytokine gene introduced thereinto and iCAR-T-mock (mock) cell not having the cytokine gene introduced thereinto were intravenously administered thereto. On day 14 after the administration of iCAR-T, the iCAR-T present in the subcutaneous tumor of each NSG mouse was collected to be analyzed by flow cytometry. Cells expressing human CD45, and mCherry that was a reporter gene incorporated into an expression vector used for the cytokine gene transfer were observed, and as a result, it was found that the amount of the iCAR-T + IL-15, the iCAR-T + IL-21, and the iCAR-T-mock present therein were 1% or less, but the amount of the iCAR-T + IL-15/21 present therein was 2.19%. In other words, it was found that the iCAR-T + IL-15/21 expressing both IL-15 and IL-21 was present in the tumor in a larger amount than the iCAR-T singly expressing IL-15 or IL-21 (Figure 15).

### Example 12

### [Single Cell RNA Sequence Analysis]

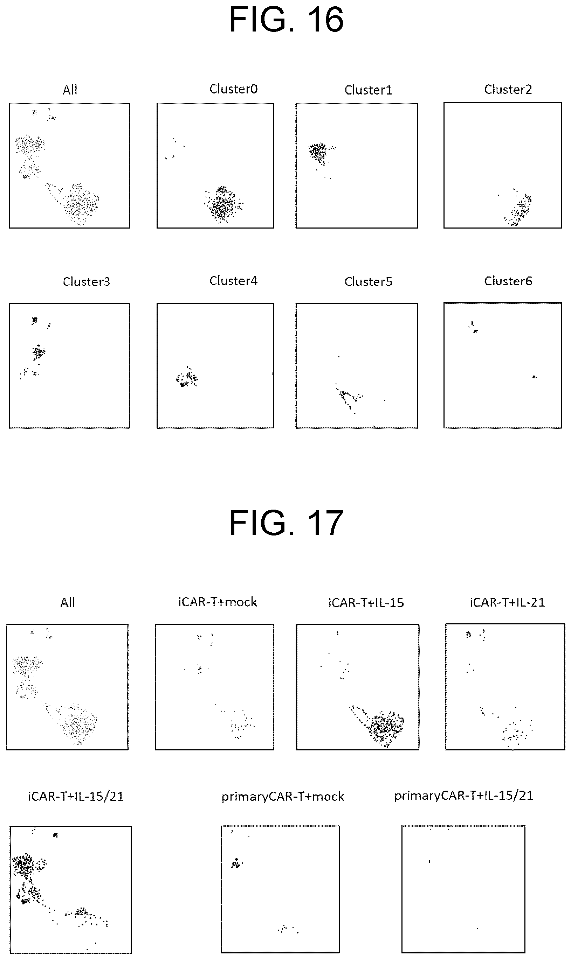

The cells analyzed in Example 11 were used to perform single cell RNA sequence analysis. Human CD45 and mCherry positive cells were sorted, and the respective individual cells were subjected to gene analysis with a next generation sequencer, and then subjected to bioinformatics analysis using Seurat (R package) (Figure 16). Thereafter, the respective analyzed data was displayed on UMAP plot. (As the positions between clusters displayed on the UMAP are farther from each other, these are cell populations more different in the gene expression profile.) Figure 17 illustrates seven clusters displayed on the UMAP, and distributions of the respective iCAR-T cells having the cytokine gene introduced thereinto. It was found based on these classifications that the iCAR-T + IL-15/21 was present in Clusters 1 and 4. On the other hand, the iCAR-T + IL-15, the iCAR-T + IL-21, and the iCAR-T-mock were found to be present in Clusters 0, 2, and 5, and were revealed to have gene expression plots that were obviously different from those of iCAR-T + IL-15/21 cells. Besides, since peripheral blood-derived T cell (primary CAR-T) was present in Cluster 3, which was disposed in a position close to Clusters 1 and 4 composed of the iCAR-T + IL-15/21, it was suggested that the gene expression profile of the iCAR-T + IL-15/21 is close to that of the peripheral blood-derived T cell. Previously, the iCAR-T has been known as an NK-cell-like cell, and has been regarded as a cell different from the peripheral blood-derived T cell. It is assumed based on the results obtained here that the iCAR-T could be made closer to the peripheral blood-derived T cell by expressing IL-15 and IL-21 in the iCAR-T. The iCAR-T + IL-15/21 has a gene expression profile different from those of the iCAR-T + IL-15, the iCAR-T + IL-21, and the iCAR-T-mock, and it was concluded that the gene expression profile was substantially the same as that of the peripheral blood-derived T cell.

### Example 13

### [Analysis of Gene Groups of Clusters 1 and 4 Composed of iCAR-T + IL-15/21]

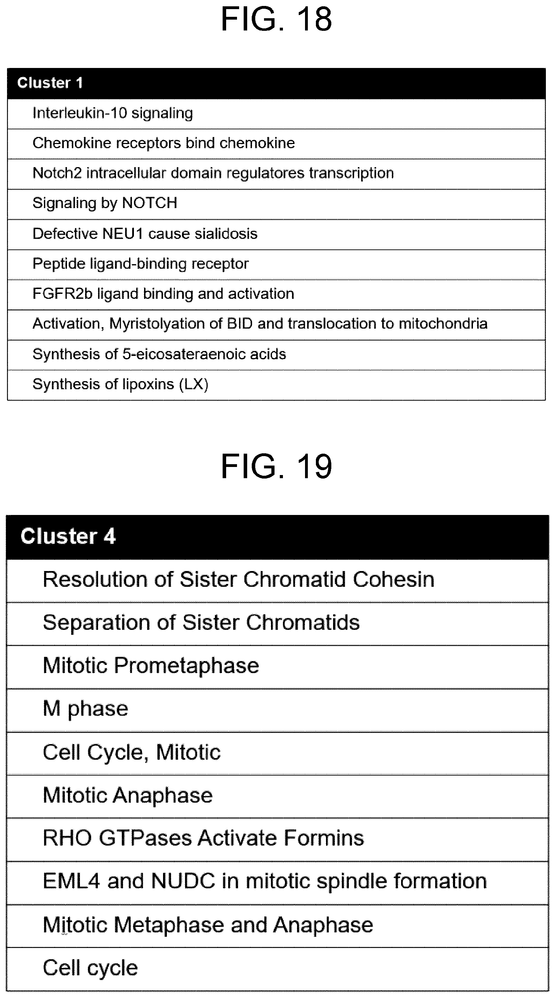

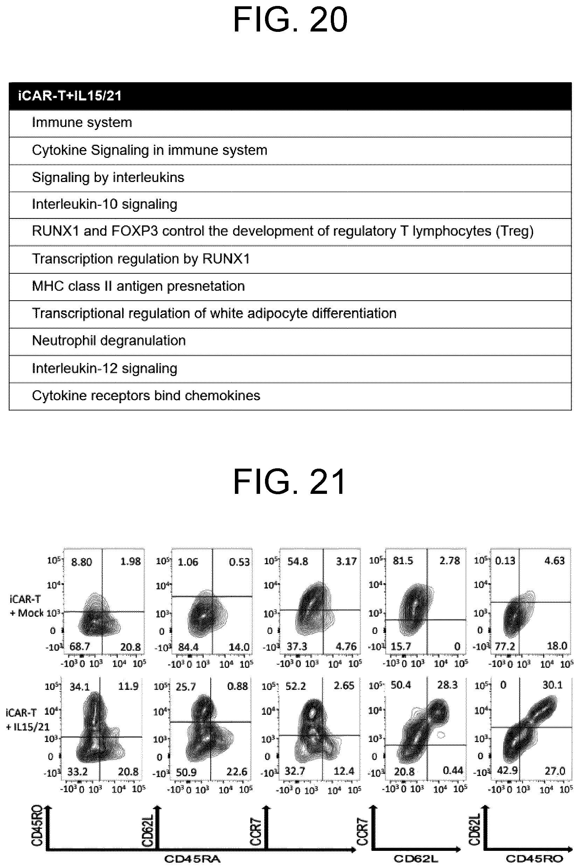

When the gene groups of Clusters 1 and 4 composed of the iCAR-T + IL-15/21 were analyzed, it was revealed that expression of chemokine-related, cytotoxic molecule and cell cycle-related genes was increased (Figures 18 and 19). A gene significantly expressed in each cluster was used for performing pathway analysis. As a result, it was found that the pathways of the chemokine-related and cell cycle-related genes were increased in Clusters 1 and 4 composed of the iCAR-T + IL-15/21. Similarly, it was found that the pathways of the immune-related and cytokine-related genes were increased in the iCAR-T + IL-15/21 (Figure 20). Although data is not shown, there was a similar trend in the peripheral blood-derived T cell.

### Example 14

### [Property of iCAR-T + IL-15/21]

The cells analyzed in Example 11 were used to observe the memory phenotypes of the respective iCAR-Ts. It is regarded that the memory phenotype changes in the order of naive, stem cell memory, central memory, and effector memory, and it is known that higher therapeutic effect is obtained in cell therapy as the number of naive-like young T cells is larger. It was found that expression of CD62L, CCR7, and CD45RO was increased in the iCAR-T + IL-15/21. T cells expressing these cell surface markers are central memory-like T cells in general. Although data is not shown, iCAR-Ts not producing IL-15 and IL-21, or expressing either IL-15 or IL-21 showed a phenotype considered as an effector memory to terminally differentiated T cell based on cell surface markers expressed therein. In conclusion, it was revealed that the iCAR-T + IL-15/21 had a young memory phenotype (Figure 21).

### Example 15

### [Enhancement of Tumor Infiltration Ability of iPS Cell-derived TCR-T Cell by IL-15 and IL-21]

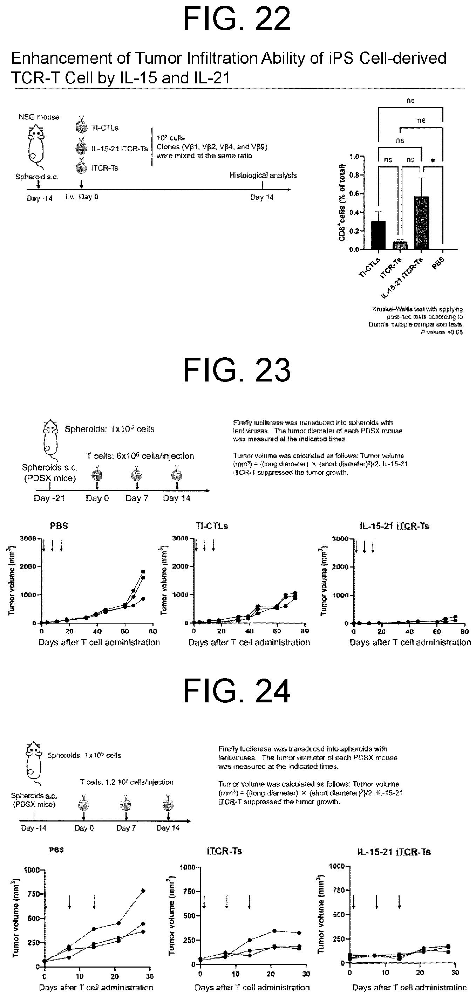

To an NSG mouse (PDSX mouse) transplanted, in the back, with colorectal cancer cell spheroids (2 × 10⁵ cells) isolated from a colorectal cancer patient, 10⁷ cells of each type of the followings were intravenously injected: A cloned T cell of a tumor infiltrating T cell isolated in establishing the colorectal cancer cell spheroid (TI-CTLs); a T cell (iTCR-Ts) induced to differentiate from an iPS cell (FF-I01s04 cell line provided by Center for iPS Cell Research and Application, Kyoto University being used) having a TCR introduced thereinto, the TCR recognizing a colorectal cancer cell spheroid, obtained from the cloned T cell; and an iTCR-Ts obtained by introducing the genes of IL-15 and IL-21 into the iTCR-Ts for constantly producing IL-15 and IL-21 (IL-15-21 iTCR-Ts). After 14 days, the tumors were collected. Each tumor tissue section was immunohistochemically stained with an anti-human CD8 antibody, and then the TI-CTLs, the iTCR-Ts, and the IL-15-21 iTCR-Ts infiltrating the tumors were analyzed. The infiltrating T cells accumulated around stromal cells and epithelial cells in the tumor tissues. The obtained results are illustrated in a graph in terms of a ratio of human CD8 cells to the total cell number (Figure 22). Although the TI-CTLs showed good tumor tissue infiltration, the iTCR-Ts showed lower infiltration ability than the TI-CTLs. On the other hand, the IL-15-21 iTCR-Ts showed tumor infiltration ability equivalent to or higher than that of the TI-CTLs owing to the production of IL-15 and IL-21 (Figure 22). It was thus clarified that an IPS cell-derived T cell having TCR introduced thereinto was definitely enhanced in tumor infiltration ability by introduction of IL-15 and IL-21, and that the degree of the enhancement was higher than that of a tumor-infiltrating T cell.

### Example 16

### [Tumor Proliferation Inhibitory Effect (I) of iPS Cell-derived TCR-T Cell by IL-15 and IL-21]

To an NSG mouse (PDSX mouse) transplanted, in the back, with colorectal cancer cell spheroids (1 × 10⁵ cells) isolated from a colorectal cancer patient and having firefly luciferase introduced thereinto, 6 × 10⁶ cells of each type of the followings were intravenously injected three times on day 0, day 7, and day 14: A cloned T cell of a tumor infiltrating T cell isolated in establishing the colorectal cancer cell spheroid (TI-CTLs); an iTCR-Ts obtained by introducing the genes of IL-15 and IL-21, for constantly producing IL-15 and IL-21, into a T cell (iTCR-Ts) induced to differentiate from an iPS cell (FF-I01s04 cell line provided by Center for iPS Cell Research and Application, Kyoto University being used) having a TCR introduced thereinto, the TCR recognizing a colorectal cancer cell spheroid obtained from the cloned T cell; and phosphate buffer (PBS). Thereafter, the proliferation of the tumors was observed over time with IVIS spectrum CT. An IL-15-21 iTCR-Ts administration group showed stronger tumor proliferation inhibitory effect than a TI-CTLs administration group (Figure 23). It was thus clarified that a TCR having iPS cell-derived T cell introduced thereinto definitely inhibited in tumor proliferation by introduction of IL-15 and IL-21, and that the degree of the inhibition was higher than that of a tumor-infiltrating T cell.

### Example 17

### [Tumor Proliferation Inhibitory Effect (II) of iPS Cell-derived TCR-T Cell by IL-15 and IL-21]

To an NSG mouse (PDSX mouse) transplanted, in the back, with colorectal cancer cell spheroids (1 × 10⁵ cells) isolated from a colorectal cancer patient and having firefly luciferase introduced thereinto, 1.2 × 10⁷ cells of each type of the followings were intravenously injected three times on day 0, day 7, and day 14: A T cell (iTCR-Ts) induced to differentiate from an iPS cell (FF-I01s04 cell line provided by Center for iPS Cell Research and Application, Kyoto University being used) having a TCR introduced thereinto, the TCR recognizing the colorectal cancer cell spheroid, obtained from the cloned T cell of a tumor infiltrating T cell isolated in establishing a colorectal cancer cell spheroid; an iTCR-Ts obtained by introducing the genes of IL-15 and IL-21 into the iTCR-Ts for constantly producing IL-15 and IL-21 (IL-15-21 iTCR-Ts); and PBS. Thereafter, the proliferation of the tumors was observed over time with IVIS spectrum CT. An IL-15-21 iTCR-Ts administration group showed stronger tumor proliferation inhibitory effect than an iTCR-Ts administration group (Figure 24). It was thus clarified that an iPS cell-derived T cell having TCR introduced thereinto definitely inhibited in tumor proliferation by introduction of IL-15 and IL-21, and that the degree of the inhibition was higher than that of an iPS cell-derived T cell not producing IL-15 and IL-21.

## Claims

1. An immune cell induced from an iPS cell, wherein the immune cell expresses a cell surface molecule reactive to a tumor-related antigen, and interleukin 15 (IL-15).

2. The immune cell according to claim 1, wherein the immune cell further expresses interleukin 12 (IL-12), interleukin 18 (IL-18), or interleukin 21 (IL-21).

3. The immune cell according to claim 1, wherein the immune cell comprises a nucleic acid encoding the cell surface molecule and a nucleic acid encoding IL-15, introduced extracellularly.

4. The immune cell according to claim 3, wherein the immune cell further comprises a nucleic acid encoding IL-12, IL-18, or IL-21, introduced extracellularly.

5. The immune cell according to claim 1, wherein the immune cell comprises an expression vector comprising a nucleic acid encoding the cell surface molecule, and a nucleic acid encoding IL-15.

6. The immune cell according to claim 5, wherein the immune cell further comprises an expression vector comprising a nucleic acid encoding IL-12, IL-18, or IL-21.

7. The immune cell according to claim 1, wherein the immune cell is a T cell or an NK cell.

8. The immune cell according to claim 7, wherein the T cell is a CD8 single positive cytotoxic T cell.

9. The immune cell according to claim 1, wherein the cell surface molecule is a chimeric antigen receptor (CAR) or a T cell receptor (TCR).

10. The immune cell according to claim 1, wherein the cell surface molecule is a chimeric antigen receptor (CAR) and a T cell receptor (TCR).

11. The immune cell according to claim 1, wherein the iPS cell is an iPS cell obtained by reprogramming a peripheral blood mononuclear cell from which a B cell and a T cell have been removed, or a T cell.

12. The immune cell according to claim 1, wherein the tumor-related antigen is selected from the group consisting of WT1, GPC3, BCMA, XAGE1, MUC1, MUCSA1, MUC6, EGFRvIII, HER-2/neu, MAGE-A1, MAGE-A3, telomerase, PRAME, SSX2/4, PSCA, CTLA-4, gp100, GD2, GD3, fucosyl GM1, GM3, sLe(a), glycolipid F77, mesothelin, PD-L1, trp1, trp2, CD19, CD20, CD22, ROR1, CD33, c-Met, p53 without gene mutation, p53 with gene mutation, a p53 mutant, NY-ESO-1, PSMA, ETV6-AML, CEA, PSA, AFP, hTERT, EpCAM, ALK, an androgen receptor, EphA2, CYP1B1, OY-TES-1, MAD-CT-2, Melan A/MART 1, survivin, Ras, a Ras mutant, EGR, bcr-ab1, XBP-1, a neo-antigen resulting from gene mutation, a neo-antigen resulting from abnormal splicing, HBV, HBs, HPV, EBV, LMP1, EBV, LMP2, EBNA, HPV-E1, HPV-E2, HPV-E6, HPV-E7, HTLV-1 Tax, and HBZ.

13. A method for producing the immune cell according to claim 3, comprising:
(1) a step of introducing a nucleic acid encoding the cell surface molecule into an iPS cell, or a hematopoietic stem cell, an immature immune cell, or a mature immune cell induced to differentiate from an iPS cell;
(2) a step of including the following step (2-1), (2-2), or (2-3) of:
(2-1) differentiating the iPS cell having the nucleic acid introduced thereinto obtained in the step (1) into a hematopoietic stem cell, an immature immune cell, or a mature immune cell;
(2-2) differentiating, into an immature immune cell or a mature immune cell, the hematopoietic stem cell induced to differentiate from the iPS cell, having the nucleic acid introduced thereinto, obtained in the step (1); or
(2-3) differentiating, into a mature immune cell, the immature immune cell induced to differentiate from the iPS cell, having the nucleic acid introduced thereinto, obtained in the step (1); and
(3) a step of introducing a nucleic acid encoding IL-15 into the mature immune cell having the nucleic acid introduced thereinto obtained in the step (1), or the hematopoietic stem cell, the immature immune cell, or the mature immune cell obtained in the step (2-1), (2-2), or (2-3).

14. A method for producing the immune cell according to claim 4, comprising:
(1) a step of introducing a nucleic acid encoding the cell surface molecule into an iPS cell, or a hematopoietic stem cell, an immature immune cell, or a mature immune cell induced to differentiate from an iPS cell;
(2) a step of including the following step (2-1), (2-2), or (2-3) of:
(2-1) differentiating the iPS cell having the nucleic acid introduced thereinto obtained in the step (1) into a hematopoietic stem cell, an immature immune cell, or a mature immune cell;
(2-2) differentiating, into an immature immune cell or a mature immune cell, the hematopoietic stem cell induced to differentiate from the iPS cell, having the nucleic acid introduced thereinto, obtained in the step (1); or
(2-3) differentiating, into a mature immune cell, the immature immune cell induced to differentiate from the iPS cell, having the nucleic acid introduced thereinto, obtained in the step (1); and
(3) a step of introducing a nucleic acid encoding IL-15, and a nucleic acid encoding IL-12, IL-18, or IL-21 into the mature immune cell having the nucleic acid introduced thereinto obtained in the step (1), or the hematopoietic stem cell, the immature immune cell, or the mature immune cell obtained in the step (2-1), (2-2), or (2-3).

15. A method for producing the immune cell according to claim 5, comprising:
(1) a step of introducing an expression vector comprising a nucleic acid encoding the cell surface molecule into an iPS cell, or a hematopoietic stem cell, an immature immune cell, or a mature immune cell induced to differentiate from an iPS cell;
(2) a step of including the following step (2-1), (2-2), or (2-3) of:
(2-1) differentiating the iPS cell having the expression vector introduced thereinto obtained in the step (1) into a hematopoietic stem cell, an immature immune cell, or a mature immune cell;
(2-2) differentiating, into an immature immune cell or a mature immune cell, the hematopoietic stem cell induced to differentiate from the iPS cell, having the expression vector introduced thereinto, obtained in the step (1); or
(2-3) differentiating, into a mature immune cell, the immature immune cell induced to differentiate from the iPS cell, having the expression vector introduced thereinto, obtained in the step (1); and
(3) a step of introducing an expression vector comprising a nucleic acid encoding IL-15 into the mature immune cell having the expression vector introduced thereinto obtained in the step (1), or the hematopoietic stem cell, the immature immune cell, or the mature immune cell obtained in the step (2-1), (2-2), or (2-3).

16. A method for producing the immune cell according to claim 6, comprising:
(1) a step of introducing an expression vector comprising a nucleic acid encoding the cell surface molecule into an iPS cell, or a hematopoietic stem cell, an immature immune cell, or a mature immune cell induced to differentiate from an iPS cell;
(2) a step of including the following step (2-1), (2-2), or (2-3) of:
(2-1) differentiating the iPS cell having the expression vector introduced thereinto obtained in the step (1) into a hematopoietic stem cell, an immature immune cell, or a mature immune cell;
(2-2) differentiating, into an immature immune cell or a mature immune cell, the hematopoietic stem cell induced to differentiate from the iPS cell, having the expression vector introduced thereinto, obtained in the step (1); or
(2-3) differentiating, into a mature immune cell, the immature immune cell induced to differentiate from the iPS cell, having the expression vector introduced thereinto, obtained in the step (1); and
(3) a step of introducing an expression vector comprising a nucleic acid encoding IL-15, and a nucleic acid encoding IL-12, IL-18, or IL-21 into the mature immune cell having the expression vector introduced thereinto obtained in the step (1), or the hematopoietic stem cell, the immature immune cell, or the mature immune cell obtained in the step (2-1), (2-2), or (2-3).

17. A pharmaceutical comprising the immune cell according to any one of claims 1 to 12.

18. The pharmaceutical according to claim 17, for use in prevention or treatment of cancer.

19. A cytotoxic agent for a cell expressing a tumor-related antigen, comprising the immune cell according to any one of claims 1 to 12.

20. A method for preventing or treating cancer in a mammal, comprising administering, to the mammal, an effective amount of the immune cell according to any one of claims 1 to 12.

21. A method for preventing or treating cancer in a mammal, comprising administering, to the mammal, an effective amount of the pharmaceutical according to claim 17.

22. A method for preventing or treating cancer in a mammal, comprising administering, to the mammal, an effective amount of the cytotoxic agent according to claim 19.

23. An agent for preventing or treating cancer in a mammal, comprising the immune cell according to any one of claims 1 to 12.

24. The immune cell according to any one of claims 1 to 12, for use in prevention or treatment of cancer.

25. The immune cell according to any one of claims 1 to 12, for producing an agent for preventing or treating cancer.
